# EUROPEAN PATENT SPECIFICATION

(11) **EP 0 356 035 B1**
(45) Date of publication and mention of the grant of the patent: **13.03.1996**
(21) Application number: 89307815.4
(22) Date of filing: 01.08.1989
(51) Int. Cl.: A61K 31/135, A61K 31/275, A61K 31/44, A61K 31/165, C07C 215/52, C07C 211/29, C07C 255/58, C07C 211/27, C07C 217/60, C07C 271/12, C07C 237/06

(54) **Arylkalkyl-amines and -amides having anticonvulsant and neuroprotective properties**
Arylalkylamine und -amide mit krampflösender und nervenschützender Wirkung
Arylalkyl-amines et -amides à activité anticonvulsive et neuroprotectrice

(30) Priority: 12.08.1988 US 232566
(43) Date of publication of application: 28.02.1990
(60) Divisional application: 94119941.6
(73) Proprietor: Astra Aktiebolag, S-151 85 Södertälje (SE)
(72) Inventor: Napier, James J., Lindenhurst Illinois 60046 (US); Griffith, Ronald C., Pittsford New York 14534 (US)
(74) Representative: England, Christopher David

(56) References cited:
- EP-A- 0 009 800
- EP-A- 0 057 870
- EP-A- 0 278 090
- EP-A- 0 279 937
- EP-A- 0 279 938
- EP-A- 0 322 582
- EP-A- 0 326 240
- WO-A-88/02254
- CH-A- 343 388
- GB-A- 916 789
- US-A- 2 503 285
- US-A- 2 691 680
- US-A- 3 510 560
- US-A- 4 757 076
- Merck Index 11th ed.

## Description

This invention relates to pharmaceutical compounds, some of which are novel, and to their anticonvulsant, sedative and neuroprotective properties.

WO-A-8802254 discloses pharmaceutical formulations including phenylethylamine derivatives and the use of such compounds in the treatment of learning deficits and depression. CH-A-343388 relates to 2-amino-N-ethyl-acetamides and their use as anticonvulsants. EP-A-0279937, EP-A-0326240, EP-A-0278090 and EP-A-0279938 relate to various 2-amino-N-ethyl-acetamides and their use as anticonvulsants. US-A-2691680 discloses N-methyl-1-(3-hydroxyphenyl)-2-phenylethylamine and its use as an analgesic. GB-A-916789 discloses pharmaceutical formulations comprising resin complexes of ethylamine derivatives. EP-B-0009800 discloses 2-amino-α-phenylphenethylamine compounds as intermediates in the preparation of benzodiazepine compounds. EP-A-0057870 claims the 2-amino-α-phenylphenethylamine compounds of EP-A-0009800 and indicates them as analgesics and anticonvulsants. EP-A-0322582 discloses α-(heterocyclyl)benzeneethanamine compounds and their use as anticonvulsants. US-A-2503285 discloses a small group of α-phenyl-β-(alkoxyhydroxyphenyl)ethylamines and their use as analgesics. US-A-3510560 discloses a large group of diphenylalkylamines which are indicated in the suppression of adrenal gland function.

Compounds which possess anticonvulsant, antihypoxic or N-methyl-(d)-aspartate (NMDA) blocking properties are useful in the treatment and/or prevention of neurodegeneration in pathological conditions such as stroke, cerebral ischaemia, cerebral palsy, hypoglycaemia, epilepsy, Alzheimer's disease, Huntington's chorea, Olivo-pontocerebellar atrophy, perinatal asphyxia and anoxia. It has now been discovered that many substituted arylalkyl-amines and -amides possess anticonvulsant and neuroprotective properties which are useful for the treatment of such disorders. Many of these compounds also possess sedative properties.

According to the invention we provide the use of a compound of formula I, 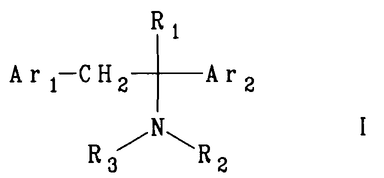
wherein
Ar₁ and Ar₂ independently represent phenyl substituted by one or more of amino, nitro, chlorine, bromine, hydroxy, C₁₋₆ alkoxy, C₁₋₆ alkyl or cyano; in addition one of Ar₁ or Ar₂ may represent phenyl;
R₁ represents hydrogen or C₁₋₆ alkyl;
R₂ represents hydrogen or COCH₂NH₂;
R₃ represents hydrogen or C₁₋₆ alkyl;
in addition, when R₂ represents hydrogen, then both of Ar₁ and Ar₂ may represent phenyl, one or both of Ar₁ and Ar₂ may represent fluorophenyl or 2-, 3- or 4-pyridinyl, and R₁ may represent C₁₋₆ alkoxycarbonyl or trifluoromethyl;
provided that:
(a) when R₁ and R₂ each represent H and Ar₁ and Ar₂ each represent phenyl, then R₃ is other than C₁₋₆ alkyl;
(b) when Ar₂ represents 2-, 3- or 4-pyridinyl, then R₁ represents C₁₋₆ alkyl; and
(c) when R₁ and R₂ each represent H, then Ar₁ does not represent phenyl substituted by amino;
or a pharmaceutically acceptable salt thereof;
as active ingredient in the manufacture of a neuroprotective or anticonvulsant medicament.

This invention also relates to all stereoisomeric forms, optical enantiomeric forms and pharmaceutically acceptable acid addition salts of the compounds of formula I.

The compounds of formula I possess useful anticonvulsant properties as demonstrated by their ability to inhibit maximal electroshock (MES) induced seizures in mice. The compounds of formula I also possess antihypoxia activity as demonstrated by their ability to increase the survival time of mice in an oxygen depleted environment. In addition, compounds of formula I inhibit the onset of convulsions and death induced by administration of N-methyl-(d)-aspartate (NMDA) to mice.

### DETAILED DESCRIPTION

Some of the compounds of formula I are known compounds and are commercially available. Compounds of formula I in which R₂ represents hydrogen may be prepared by known procedures for amine formation, for example those given in "Advanced Organic Chemistry", 2^{nd} Ed., J March, (McGraw-Hill) at page 1172. Compounds of formula I in which R₂ represents COCH₂NH₂ may be prepared by known procedures for amide formation, for example those given in "Advanced Organic Chemistry" at page 1171.

Methods for the preparation of compounds of formula I are also given in US Patents N°s 4,769,466 and 4,798,687.

Certain compounds of formula I are novel, thus according to a further aspect of the invention we provide a compound of formula IA, 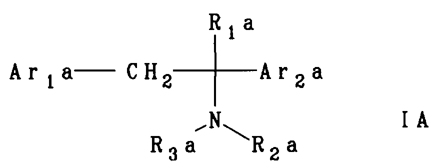
wherein
Ar₁a and Ar₂a independently represent phenyl substituted by one or more of amino, nitro, chlorine, bromine, hydroxy, C₁₋₆ alkoxy, C₁₋₆ alkyl or cyano; in addition one of Ar₁a or Ar₂a may represent phenyl;
R₁a represents hydrogen or C₁₋₆ alkyl;
R₂a represents hydrogen or COCH₂NH₂;
R₃a represents hydrogen or C₁₋₆ alkyl;
provided that when R₂a represents hydrogen, then R₁a represents C₁₋₆ alkyl; or a pharmaceutically acceptable salt thereof.

The invention further provides a process for the preparation of a compound of formula IA, as defined above, or a pharmaceutically acceptable salt thereof, which comprises:
a) for compounds in which R₂a represents hydrogen and R₃a represents C₁₋₆ alkyl, alkylation of a corresponding compound of formula IA in which R₃a represents hydrogen;
b) for compounds in which R₂a and R₃a both represent hydrogen, amide hydrolysis of a corresponding compound of formula IIA, 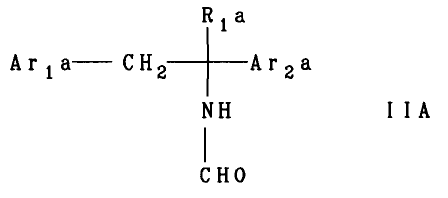 in which Ar₁a, Ar₂a and R₁a are as defined above;
c) for compounds in which R₂a and R₃a both represent hydrogen, reductive carbamate cleavage of a compound of formula IIIA, 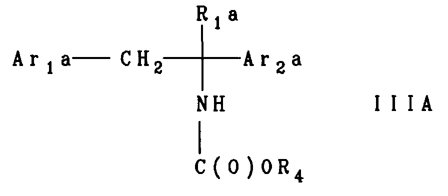 in which Ar₁a, Ar₂a and R₁a are as defined above and R₄ represents an alkyl or aryl group;
d) for compounds in which R₂a represents COCH₂NH₂, removal of the amine protecting group from a compound of formula IVA, 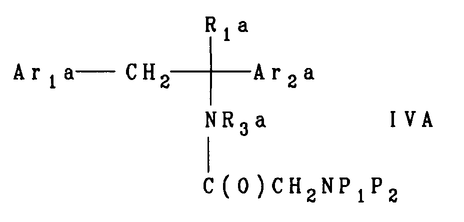 in which P₁ and P₂ together constitute a suitable protecting group, and Ar₁a, Ar₂a, R₁a and R₃a are as defined above; or
e) for compounds in which R₂a represents COCH₂NH₂, aminating a compound of formula VA, 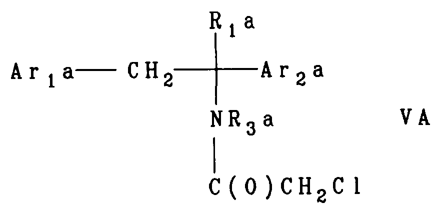 in which Ar₁a, Ar₂a, R₁a and R₃a are as defined above.

Analogous processes to those described above may be used for the preparation of other compounds of formula I.

Many alkylation procedures may be used for the reaction of method a), for example a primary amine of formula IA in which R₂a and R₃a both represent hydrogen may be reacted with a suitable alkyl halide, for example the alkyl chloride, in an inert solvent such as tetrahydrofuran, in the presence of a base such as triethylamine.

The hydrolysis of method b) may be acid or base catalysed, for example the compound may be treated with 10% hydrochloric acid, and heated at reflux. Compounds of formula IIA may be prepared by reacting a compound of formula VIA; 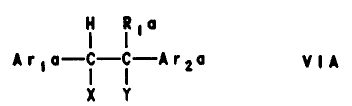
in which Ar₁a, Ar₂a and R₁a are as defined above, and either X represents hydrogen and Y represents OH, or X and Y together form a second bond between the carbons to which they are attached with cyanide ion using conditions under which the nitrogen acts as the nucleophile rather than the carbon. For example, the compound of formula VIA may be added to a mixture prepared by adding concentrated sulphuric acid to a suspension of sodium cyanide in glacial acetic acid and n-butylether. The isocyanide thus formed is hydrolysed to the corresponding isonitrile during work-up, upon addition of the reaction mixture to ice. Compounds of formula VIA are either commercially available, or may be made from commercially available compounds using known methods.

The reductive cleavage of method c) may be carried out using catalytic hydrogenation conditions, for example using 10%Pd/C as the catalyst and a 1:1 mixture of an alcohol such as methanol and 3N HCl as solvent at a pressure of about 2.7 atm [40psi] hydrogen. Alternatively, reducing agents such as zinc dust in a solvent such as 1:9 tetrahydrofuran: acetic acid may be used. Compounds of formula IIIA may be prepared by Curtius rearrangement of a corresponding compound of formula VIIA: 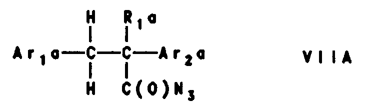
in which Ar₁a, Ar₂a and R₁a are as defined above, in the presence of an alcohol of formula R₄aOH, where R₄a represents an alkyl or aryl group, for example 2,2,2-trichloroethanol or benzyl alcohol. Compounds of formula VIIA may be prepared by reacting the corresponding acid with diphenylphosphorylazide in a solvent such as toluene in the presence of a base such as triethylamine. These corresponding acids are either commercially available or may be prepared from commercially available materials using known methods.

For method d), suitable protecting groups that P₁ and P₂ may together constitute include: a urethane protecting group such as benzyloxycarbonyl (CBZ) or t-butyloxycarbonyl (BOC); or P₁ and P₂ together with the nitrogen atom to which they are attached may form a phthalimide group. The amine protecting groups may be removed by either catalytic hydrogenation for the CBZ group, a suitable catalyst being palladium or platinum on carbon, and the reaction being suitably carried out in an inert solvent such as methanol; treatment with an acid such as trifluoroacetic or hydrochloric acid for the BOC group; or treatment with hydrazine in a lower alkanol such as ethanol for the phthalimide group. Compounds of formula IVA may be prepared by reacting a compound of formula IA in which R₂a represents hydrogen with a compound of formula VIIIA in which P₁ and P₂ are as defined above: 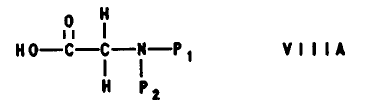
This reaction may be carried out in an inert solvent, such as tetrahydrofuran, in the presence of a coupling reagent such as dicyclohexylcarbodiimide with or without 1-hydroxybenzotriazole or other additives. Compounds of formula VIIIA are commercially available or may be made by known methods.

For method e), the amination reaction may be carried out by reacting a compound of formula VA with ammonia in a solvent such as a lower alkanol, for example methanol or ethanol, or a chlorinated solvent, for example chloroform or methylene chloride, or mixtures thereof. Compounds of formula VA may be prepared by reacting a compound of formula IA in which R₂a represents hydrogen with an activated two carbon acid derivative which contains a leaving group α to the carbonyl, such as chloroacetyl chloride, in the presence of an acid acceptor, such as triethylamine.

A group of compounds of formula IA which may be specifically mentioned is that in which R₁a, R₂a and R₃a are as defined above, and either one or both of Ar₁a and Ar₂a represents 4-hydroxyphenyl.

We prefer that Ar₁a and Ar₂a represent unsubstituted phenyl. When Ar₁a and Ar₂a are substituted, we prefer that they are mono- or disubstituted.

Where R₁a or R₃a or a substituent on an aromatic ring represents alkyl or alkoxy, we prefer that it contains up to 4 carbon atoms, for example ethyl, propyl and especially methyl. R₁a is preferably hydrogen or, more preferably, methyl. R₃a is preferably hydrogen.

Compounds of formula I which are useful as neuroprotectives or anticonvulsants include:
1,2-diphenylethylamine
1,2-diphenyl-2-propylamine;
1,2-bis(4-fluorophenyl)-2-propylamine;
1,2-diphenyl-2-butylamine;
(-)-1,2-diphenyl-2-propylamine;
(+)-1,2-diphenyl-2-propylamine;
2,3-diphenyl-2-aminopropanoic acid methyl ester;
N-methyl-1,2-diphenyl-2-propylamine;
1-(3-nitrophenyl)-2-phenyl-2-propylamine;
1-(3-chlorophenyl)-2-phenyl-2-propylamine;
1-(3-bromophenyl)-2-phenyl-2-propylamine;
1-(3-cyanophenyl)-2-phenyl-2-propylamine;
2-(2-methylphenyl)-1-phenyl-2-propylamine;
1-(4-chlorophenyl)-2-phenyl-2-propylamine;
1-phenyl-2-(3,4-dichlorophenyl)-2-propylamine;
1-phenyl-2-(3-methoxyphenyl)-2-propylamine;
1-(4-hydroxyphenyl)-2-phenyl-2-propylamine;
1-(4-hydroxyphenyl)-2-phenylethylamine;
1-phenyl-2-(4-hydroxyphenyl)ethylamine;
1,2-bis(4-hydroxyphenyl)ethylamine;
1-phenyl-2-(4-hydroxyphenyl)-2-propylamine;
1,2-bis(4-hydroxyphenyl)-2-propylamine;
1-phenyl-2-(2-pyridinyl)ethylamine;
1-phenyl-2-(3-pyridinyl)ethylamine;
1-phenyl-2-(4-pyridinyl)ethylamine;
3,3,3-trifluoro-1,2-diphenyl-2-propylamine;
N-methyl-3,3,3-trifluoro-1,2-diphenyl-2-propylamine.

The compounds of general formula I are basic compounds and may be used as such or pharmaceutically acceptable acid addition salts may be prepared by treatment with various inorganic or organic acids, such as hydrochloric, hydrobromic, sulphuric, phosphoric, acetic, lactic, succinic, fumaric, malic, maleic, tartaric, citric, benzoic, methanesulphonic or carbonic acids.

For the above mentioned uses the dosage administered will, of course, vary with the compound employed, the mode of administration and the treatment desired. However, in general, satisfactory results are obtained when the compounds are administered at a daily dosage of from about 0.1 mg to about 20 mg per kg of animal body weight, preferably given in divided doses 1 to 4 times a day or in sustained release form. For man the total daily dose is in the range of from 5 mg to 1,400 mg more preferably from 10 mg to 100 mg, and unit dosage forms suitable for oral administration comprise from 2 mg to 1,400 mg of the compound admixed with a solid or liquid pharmaceutical carrier or diluent.

The compounds of formula I, and pharmaceutically acceptable derivatives thereof, may be used on their own or in the form of appropriate medicinal preparations for enteral or parenteral administration.

According to the invention there is also provided a pharmaceutical composition comprising preferably less than 80% and more preferably less than 50% by weight of a compound of formula I, or a pharmaceutically acceptable salt thereof, in admixture with a pharmaceutically acceptable adjuvant, diluent or carrier.

Examples of such adjuvants, diluents and carriers are: for tablets and dragees: lactose, starch, talc, stearic acid; for capsules: tartaric acid or lactose; for injectable solutions: water, alcohols, glycerin, vegetable oils; for suppositories: natural or hardened oils or waxes.

Compositions in a form suitable for oral, i.e. oesophageal administration include tablets, capsules and dragees;
sustained release compositions include those in which the active ingredient is bound to an ion exchange resin which is optionally coated with a diffusion barrier to modify the release properties of the resin.

We prefer the composition to contain up to 50% and more preferably up to 25% by weight of the compound of formula I, or of the pharmaceutically acceptable derivative thereof.

The compounds of formula I and pharmaceutically acceptable derivatives thereof have the advantage that they are less toxic, more efficacious, are longer acting, have a broader range of activity, are more potent, produce fewer side effects, are more easily absorbed or have other useful pharmacological properties, than compounds of similar structure.

The compounds of general formula I possess useful pharmaceutical properties. In particular they possess useful antiepileptic properties, antihypoxia activities and/or NMDA blocking activities. These activities were assessed by standard methods.

Antiepileptic activity was measured by assessing a compound's ability to prevent the hind limb tonic extension component of the seizure in groups of mice induced by maximal electroshock (MES) after oral or intraperitoneal administration according to the procedures of the Epilepsy Branch, NINCDS as published by R. J. Porter, et al, Cleve. Clin. Quarterly 1984, 51, 293, and compared to the standard agents dilantin and phenobarbital. Activities (ED₅₀'s) in the range of 10-400 mg/kg after oral administration in this assay system were obtained.

The compounds of this invention possess useful antihypoxia activity, that is, they extend the lifetime of animals exposed to a hypoxic environment. This activity is conveniently measured in mice. Groups of mice are tested at various times after the intraperitoneal administration of graded doses of the test compound. The animals' survival time in a temperature controlled hypoxic environment (96% nitrogen and 4% oxygen) is recorded. A statistical comparison is made between coincident vehicle treated animals and the experimental group. The dose-response and minimum active dose (MAD) for compounds are obtained. Other modes of administration can also be used.

NMDA blocking activity was measured by assessing a compound's ability to protect mice from convulsions induced by intravenous administration of 150 mg/kg of NMDA according to the procedures of Czuczwar et al., (Neurotransmitters, Seizures and Epilepsy III, edited by G. Nistico et al., Raven Press, New York 1986, pages 235-246). Groups of mice were pretreated by 30 min with the test compound by the oral or intraperitoneal routes and then given NMDA. Animals were observed for convulsions as defined by loss of righting reflex. Animals were kept for 60 min after NMDA dosing and mortality was recorded.

NMDA and glycine receptor affinity was also tested in the [³H]L-glutamate and [³H]glycine binding assays following the method of Monaghan & Cotman, PNAS, 83, 7532. (1986) and Watson et al, Neurosci. Res. Comm., 2, 169, (1988).

The following in vitro methods are also used to measure NMDA blocking activity: NMDA (20µM) is applied to tissue slices of rat hippocampus (450µm thick) for approximately 2 minutes causing a massive depolarisation of hippocampal neurons, and a profound reduction of the synaptic field potential. The test is repeated several times to obtain a base line response. The compound under investigation is then included in the buffer bathing the slice, and NMDA is reapplied. The ability of the compound to block the reduction in field potential produced by NMDA is then determined.

In a second in vitro assay, rat hippocampal slices (450µm thick) are pretreated with a buffer containing 10µM 6,7-dinitroquinoxaline-2,3-dione (DNQX) and magnesium (25µM). Under these conditions the synaptic response is almost entirely mediated by NMDA receptors. To evaluate NMDA antagonism, the test compounds are added to the buffer and the synaptic field potentials are compared before and during treatment. The decrease in response caused by the drug is expressed as a percentage of the pre-drug response.

The following compounds of formula I or their acid salts were either commercially available or prepared as described in the aforementioned literature:
1,2-diphenyl-2-propylamine
1,2-bis(4-fluorophenyl)-2-propylamine
1,2-diphenyl-2-butylamine
(-)1,2-diphenyl-2-propylamine
(+)1,2-diphenyl-2-propylamine
2,3-diphenyl-2-aminopropanoic acid methyl ester
N-methyl-1,2-diphenyl-2-propylamine
1-phenyl-2-(2-pyridinyl)ethylamine
1-phenyl-2-(3-pyridinyl)ethylamine
1-phenyl-2-(4-pyridinyl)ethylamine
3,3,3-trifluoro-1,2-diphenyl-2-propylamine
N-methyl-3,3,3-trifluoro-1,2-diphenyl-2-propylamine
Additional examples of the compounds of formula I were prepared as described below.

### Example 1

### Preparation of 1-(4-Hydroxyphenyl)-2-phenyl-2-propylamine hydrochloride

### a) Preparation of 1-[4-(Phenylmethoxy)phenyl]-2-phenyl-2-propylamine maleate

To a stirred suspension of α-methyl-α-[[4-(phenylmethoxy)phenyl]-methyl]benzeneacetic acid (12.9 g, 0.372 mol), prepared by the reaction of the dilithium salt of 2-phenylpropionic acid with 4-(phenylmethoxy)benzyl bromide in benzene (200 ml) under nitrogen were added triethylamine (5.2 ml, 0.037 mol) and diphenylphosphoryl azide (8.4 ml, 0.037 mol) and the solution was heated to reflux for 2 hours. To the reaction was added 2,2,2-trichloroethanol (17.4 ml, 0.18 mol) and the reaction was heated at reflux for 20 h. The reaction mixture was cooled to ambient, diluted with ethyl acetate (100 ml), washed with water (75 ml), 1N HCl (75 ml), 1N NaOH (75 ml), saturated NaCl (100 ml), dried over magnesium sulphate, and the solvent removed to provide 40.2 g of an oil. This oil was dissolved in tetrahydrofuran (100 ml) and 90% acetic acid (200 ml), and the solution cooled to 5°C. Zinc dust (60 g, 0.92 mol) was added, the mixture was warmed to ambient temperature and stirred overnight. The reaction mixture was filtered and the filtrate was concentrated to an oil. This oil was dissolved in ethyl acetate (200 ml) and water (100 ml), basified with solid sodium carbonate, filtered, the phases separated, and the aqueous phase extracted with ethyl acetate (200 ml). The combined ethyl acetate extracts were washed with saturated NaCl, dried over magnesium sulphate, and the solvent removed to provide 13.1 g of an oil. This oil was purified by silica gel chromatography on a Waters Prep 500, eluting with ammoniated 3%-methanol/methylene chloride to provide 10.6 g of an oil. To a solution of this oil (4.0 g) in ethyl acetate (50 ml) was added maleic acid (1.5 g, 0.013 mol). The solid which formed was isolated by filtration and vacuum dried to provide 2.9 g of 1-[4-(phenylmethoxy)phenyl]-2-phenyl-2-propylamine maleate, mp 180-181°C.

### b) Preparation of 1-(4-Hydroxyphenyl)-2-phenyl-2-propylamine hydrochloride

To a solution of 1-[4-(phenylmethoxy)phenyl]-2-phenyl-2-propylamine (4.2g, 0.013 mol) in methanol (200 ml), 1N hydrochloric acid (50 ml) and tetrahydrofuran (50 ml) was added 5% palladium on carbon (0.9 g). The mixture was shaken on a Parr apparatus under a pressure of 241-276 kPa [35-40 psi] of hydrogen for 16 h. The catalyst was removed by filtration and the majority of the solvent removed under vacuum. The residue was basified with 1N sodium bicarbonate (100 ml) and extracted with chloroform (4x100 ml). The combined chloroform extracts were washed with saturated sodium chloride (75 ml) and dried over magnesium sulphate. Removal of solvent gave 3.0 g of an oil. This oil was dissolved in methanol (60 ml), acidified with gaseous HCl and diluted with ether (120 ml). The solid which formed was isolated by filtration and vacuum dried at 70°C for 60 hours to provide 1.6 g of 1-(4-hydroxyphenyl)-2-phenyl-2-propylamine hydrochloride; mp 247-248°C.

### Example 2

### Preparation of 1-(4-Hydroxyphenyl)-2-phenylethylamine hydrochloride

### a) Preparation of 2-Phenyl-1-[4-(phenylmethoxy)phenyl]ethylamine hydrochloride

To a solution of lithium bis(trimethylsilyl)amide (0.047 mol) in tetrahydrofuran (80 ml) and hexane (30 ml) at 0°C under nitrogen was added a solution of 4-(phenylmethoxy)benzaldehyde (10.0 g, 0.047 mol). The solution was stirred at approximately 4-10°C for 45 min and a solution of benzylmagnesium chloride (23 ml of 2M THF solution, 0.046 mol) was added. The solution was allowed to warm to ambient temperature and stirred at that temperature overnight. The solution was cooled in an ice-water bath and saturated ammonium chloride (5.7 ml) was added. The precipitate solid was removed by filtration and the filtrate concentrated to give 13.9 g of an oil. This was dissolved in 2-propanol (50 ml) and acidified with gaseous HCl. The white solid which formed was isolated by filtration and dried to give 11.78 g of 2-phenyl-1-[4-(Phenylmethoxy)phenyl]ethylamine hydrochloride; mp 203-204°C.

### b) Preparation of 1-(4-Hydroxyphenyl)-2-phenylethylamine hydrochloride

To a solution of 1-[4-(phenylmethoxy)phenyl]-2-phenylethylamine (4.5 g, 0.013 mol) in methanol (200 ml) and 1N hydrochloric acid (50 ml) was added 5% palladium on carbon (0.8 g). The mixture was shaken on a Parr apparatus under a pressure of 241-276 kPa [35-40 psi] of hydrogen for 3 h. The catalyst was removed by filtration, and the filtrate concentrated under vacuum to give a white solid. This solid was recrystallised from 2-propanol (75 ml) and ether (50 ml) and vacuum dried at 80°C for 48 h to give 2.31 g of 1-(4-hydroxyphenyl)-2-phenylethylamine hydrochloride; mp 203-204°C.

### Example 3

### Preparation of 1-Phenyl-2-(4-hydroxyphenyl)ethylamine maleate

### a) Preparation of 1-Phenyl-2-[4-(phenylmethoxy)phenyl]ethylamine hydrochloride

By procedures essentially the same as those described in Example 1a the corresponding 1-phenyl-2-[4-(phenylmethoxy)phenyl]ethylamine hydrochloride 209-210°C, was prepared.

### b) Preparation of 1-Phenyl-2-(4-hydroxyphenyl)ethylamine maleate

To a solution of 1-pheny1-2-[4-(phenylmethoxy)phenyl]ethylamine (4.2 g, 0.012 mol) in methanol (150 ml) and 1N hydrochloric acid (40 ml) was added 5% palladium on carbon (0.5 g). The mixture was shaken on a Parr apparatus under a pressure of 276 kPa [40 psi] of hydrogen for 18 h. The catalyst was removed by filtration, and the filtrate was concentrated under vacuum to give a white solid. Two recrystallizations from 2-propanol, methanol and ether gave 3.68 g of a white solid. The above solid (3.3 g) was partitioned between 1N NaHCO₃ and a mixture of chloroform, methylene chloride and methanol (50-50-15 ml). The aqueous solution was extracted with methylene chloride (2x100 ml). The combined organic extracts were washed with saturated sodium chloride (75 ml) and dried over magnesium sulphate. Removal of solvent gave 2.3 g of a white solid. The above solid was treated with maleic acid (1.3 g) in ethyl acetate/methanol to provide 2.07 g of 1-phenyl-2-(4-hydroxyphenyl)ethylamine maleate; mp 181-182°C.

### Example 4

### Preparation of 1,2-Bis(4-hydroxyphenyl)ethylamine fumarate

### a) Preparation of 1,2-Bis[4-(phenylmethoxy)phenyl]ethylamine hydrochloride

By procedures essentially the same as those described in Example 1a, the corresponding 1,2-bis[4-(phenylmethoxy)phenyl]ethylamine hydrochloride, mp 185-187°C, was prepared.

### b) Preparation of 1,2-Bis(4-hydroxyphenyl)ethylamine fumarate

To a solution of 1,2-bis[4-(phenylmethoxy)phenyl]ethylamine (9.60 g, 0.0216 mol) in methanol (300 ml) and 1N hydrochloric acid (30 ml) was added 5% palladium on carbon (1.1 g). The mixture was shaken on a Parr apparatus under a pressure of 2.7 atm [40 psi] of hydrogen for 17 h. The catalyst was removed by filtration through celite, and the filtrate was concentrated under vacuum to give 5.6 g of a white solid. The above solid was recrystallised from methanol, isopropanol and ether to give 4.62 g of a white solid.

To a suspension of the above solid (3.6 g) in water (50 ml) were added 1N sodium bicarbonate (50 ml), ethyl acetate (100 ml) and methanol (5 ml). The phases were separated and the aqueous phase was extracted with ethyl acetate (2x100 ml). The combined organic extracts were washed with saturated sodium chloride (50 ml) and dried over magnesium sulphate. Removal of solvent gave 2.6 g of a white solid. The above solid was dissolved in ethyl acetate (80 ml) and methanol and treated with maleic acid. The solution was diluted with ethyl acetate (30 ml), concentrated to a volume of 40 ml, and again diluted to a volume of 80 ml with ethyl acetate to provide a white solid. This solid was vacuum dried at 60°C for 72 h to provide 2.6 g of 1,2-bis(4-hydroxyphenyl)ethylamine fumarate, mp 275-277°C (D).

### Example 5

### Preparation of 1-Phenyl-2-(4-hydroxyphenyl)-2-propylamine hydrochloride

### a) Preparation of 1-Phenyl-2-[4-(phenylmethoxy)phenyl]-2-propylamine maleate

By procedures essentially the same as those described in Example 1a, the corresponding 1-phenyl-2-[4-(phenyl-methoxy)phenyl]-2-propylamine maleate, mp 154-155°C, was prepared.

### b) Preparation of 1-Phenyl-2-(4-hydroxyphenyl)-2-propylamine hydrochloride

To a solution of 1-phenyl-2-[4-(phenylmethoxy)phenyl)-2-propylamine (3.50 g, 0.011 mol) in methanol (100 ml), tetrahydrofuran (50 ml) and 1N hydrochloric acid (20 ml) was added 5% palladium on carbon (0.8 g) and the mixture was shaken on a Parr apparatus at approximately 276 kPa [40 psi] for 6 h. The catalyst was removed by filtration and the solvent removed under vacuum. Crystallization from 2-propanol and ether and vacuum drying at 80°C for 48 h provided 1.3 g of 1-phenyl-2-(4-hydroxyphenyl)-2-propyl amine hydrochloride; mp 160-161°C.

### Example 6

### Preparation of 1,2-Bis(4-hydroxyphenyl)-2-propylamine acetate

### a) Preparation of 1,2-Bis[4-(phenylmethoxy)phenyl]-2-propylamine fumarate

By a procedure essentially the same as that described in Example 1a, the corresponding 1,2-bis[4-(phenylmethoxy)phenyl]-2-propylamine fumarate, mp 161-163°C, was prepared.

### b) Preparation of 1,2-Bis(4-hydroxyphenyl)-2-propylamine acetate

To a solution of 1,2-bis[4-(phenylmethoxy)phenyl]-2-propylamine (4.56 g, 0.011 mol) in tetrahydrofuran (200 ml), and acetic acid (50 ml) was added 5% palladium on carbon (0.8 g). The mixture was shaken on a Parr apparatus in an atmosphere of 241-276 kPa [35-40 psi] of hydrogen for 16 h. The catalyst was removed by filtration, and the filtrate concentrated under vacuum to provide an oil (6.6 g). Crystallization of this oil from ethyl acetate and methanol and vacuum drying at 60°C for 48 h provided 1.80 g of 1,2-bis(4-hydroxyphenyl)-2-propylamine acetate; mp 157-159°C.

### Example 7

### Preparation of 1-(3-Nitrophenyl)-2-phenyl-2-propylamine fumarate

To a suspension of sodium cyanide (34.3g, 0.7mol) in glacial acetic acid (500ml) and n-butylether (100ml) at 0°C was added portionwise concentrated sulphuric acid (200ml). The ice bath was removed and a solution of 1-(3-nitrophenyl)-2-phenyl-1-propene (prepared from [(3-nitrophenyl)methylene]triphenylphosphorane and acetophenone, 0.5mol) in n-butylether (100ml) was added dropwise over a period of 2 hours, then the mixture stirred for 48 hours. The mixture was poured into 1000ml ice, and extracted with chloroform. The extracts were washed with water, dried and evaporated to a solid residue which was stirred with hexane (500ml), filtered and dried to give N-formyl-1-(3-nitrophenyl)-2-phenyl-2-propylamine. This was dissolved in methanol (100ml) and 1N HCl (100ml) was added. This mixture was heated to 66-60°C for 24 hours then the solvents were removed and the residue was suspended in 10% NaOH (500ml) and extracted with chloroform (3x300ml). The combined chloroform extracts were dried over magnesium sulphate and the solvent removed to leave an oil. This oil was treated with fumaric acid in ethyl acetate/ isopropanol to give the title compound in 97% yield. Mp 234-235°C.

### Example 8

### Preparation of 1-(3-chlorophenyl)-2-phenyl-2-propylamine maleate

To a solution of N-formyl-1-(3-nitrophenyl)-2-phenyl-2-propylamine (5.0g, 0.017mol) in methanol (200ml) was added 10% Pd/C catalyst (0.5g) and the mixture hydrogenated at 345 kPa [50psi] in a Parr apparatus for 3 hours. The catalyst was removed by filtration and the solvent evaporated to a white solid, 4.6g. This solid was recrystallised from isopropanol (50ml) to give 2.6g of N-formyl-1-(3-aminophenyl)-2-phenyl-2-propylamine, mp 114-115°C. To a stirred solution of sodium nitrate (2.0g, 0.03mol) in sulphuric acid (15ml) at 0°C under nitrogen was added dropwise a solution of N-formyl-1-(3-aminophenyl)-2-phenyl-2-propylamine (7.0g, 0.027mol) in acetic acid (75ml) and the solution was stirred 2.5 hours at a temperature less than 20°C. The above mixture was added to a solution of cuprous chloride (5.3g, 0.054mol) in concentrated HCl (50ml) at 15°C, and the resulting solution was stirred at that temperature for 1 hour. The reaction mixture was poured into water (500ml), basified with concentrated ammonium hydroxide, and extracted with chloroform (3x200ml). The combined chloroform extracts were dried over magnesium sulphate and the solvent evaporated to a solid, 7.5g. This solid was recrystallised from isopropanol (20ml) to provide 3.2g of N-formyl-1-(3-chlorophenyl)-2-phenyl-2-propylamine, mp 108-109°C.

To a stirred solution of N-formyl-1-(3-chlorophenyl)-2-phenyl-2-propylamine (5.5g, 0.017mol) in methanol (100ml) was added 1N HCl (100ml) and the mixture was heated to 55-60°C for 24 hours. The solvents were removed, the residue suspended in 10% NaOH (500ml), and extracted with chloroform (3x300ml). The combined chloroform extracts were dried over magnesium sulphate and the solvent removed to provide 4.3g of an oil. This oil was treated with maleic acid in ethyl acetate to provide the title compound, mp 151-152°C.

### Example 9

### Preparation of 1-(3-Bromophenyl)-2-phenyl-2-propylamine maleate

By procedures essentially the same as those described in Example 8, and by substituting cuprous bromide for cuprous chloride and 48% HBr for conc. HCl, the title compound was prepared. Mp 148-149°C

### Example 10

### Preparation of 1-(3-Cyanophenyl)-2-phenyl-2-propylamine maleate

By procedures essentially the same as those described in Example 8, and by substituting cuprous cyanide for cuprous chloride and water for conc. HCl, the title compound was prepared. Mp 157-158°C.

### Example 11

### Preparation of 2-(2-Methylphenyl)-1-phenyl-2-propylamine fumarate

To a stirred solution of o-tolylacetic acid (50.0g, 0.33mol) in tetrahydrofuran (400ml) and hexamethyl-phosphoric triamide (116ml, 0.668mol) at 0°C was added n-butyllithium (416ml of 1.6M hexane solution, 0.666mol). The solution was warmed to ambient temperature and stirred for 0.5 hour. The solution was cooled to -78°C and iodomethane (20.7ml, 0.33mol) was added and the reaction allowed to warm to room temperature and stirred for 45 minutes. The reaction mixture was cooled to 0°C and n-butyllithium (208ml of 1.6M hexane soltion, 0.333mol) was added. The reaction was warmed to ambient temperature for 10 minutes, recooled to 0°C and benzyl bromide (40.4ml, 0.333mol) was added. The reaction mixture was warmed to ambient temperature and stirred at that temperature overnight. The reaction was poured into 1N HCl and extracted with ethyl acetate. The organic solution was washed with water (2x) saturated sodium chloride, and dried over magnesium sulphate. Removal of the solvent gave 111g of an oil. To a solution of the above oil (111g) in toluene (600ml) were added triethylamine (51ml, 0.36mol) and diphenylphosphoryl azide (82.5ml, 0.38mol) and the solution was heated to reflux under nitrogen for 2 hours. Benzyl alcohol (138ml, 1.3mol) was added and the solution was refluxued overnight. The reaction was cooled to ambient temperature, diluted with ethyl acetate (300ml), washed with 1N HCl (2x), 5% sodium hydroxide (2x), saturated sodium chloride, dried over magnesium sulphate, and the solvent removed to provide 300g of an oil. The above oil (300g) was dissolved in methanol (1.81) and 3N HCl (200ml) and hydrogenated at 276 kPa [40psi] of hydrogen over 10% Pd/C (17.4g) for 2 hours. The catalyst was removed by filtration and the solvents evaporated. The residue was dissolved in chloroform (300ml) and washed with 1N sodium carbonate (2x300ml), saturated sodium chloride and dried over magnesium sulphate. Removal of solvent gave an oil which was purified by silica gel chromatography, elution with ammoniated 25% ethyl acetate-hexane, to provide 14.5g of 2-(2-methylphenyl)-1-phenyl-2-propylamine as an oil. The above oil (0.5g) was treated with fumaric acid in ethyl acetate/isopropanol to provide 0.35g of the title compound, mp 180-182°C.

### Example 12

### Preparation of 1-(4-Chlorophenyl)-2-phenyl-2-propylamine maleate

By procedures essentially the same as those described in Example 8, the title compound was prepared, mp 174.5-175.5°C.

### Example 13

### Preparation of 1-Phenyl-2-(3,4-Dichlorophenyl)-2-propylamine maleate

By procedures essentially the same as those described in Example 8, the title compound was prepared, mp 164-165°C.

### Example 14

### Preparation of 1-Phenyl-2-(3-methoxyphenyl)-2-propylamine maleate

By procedures essentially the same as those described in Example 11, the title compound was prepared, mp 139-140°C.

### Example 15

### Preparation of 2-(3-Chlorophenyl)-1-phenyl-2-propylamine maleate

By procedures essentially the same as those described in Example 8, the title compound was prepared. Mp 174-175°C.

### Example 16

### Preparation of 1-(2-Chlorophenyl)-2-phenyl-2-propylamine maleate

By procedures essentially the same as those described in Example 8, the title compound was prepared. Mp 169-171°C.

### Example 17

### Preparation of 2-(2-Chlorophenyl)-1-phenyl-2-propylamine fumarate

By procedures essentially the same as those described in Example 8, the title compound was prepared. Mp 191-193°C.

### Example 18

### Preparation of 2-(3-Nitrophenyl)-1-phenyl-2-propylamine maleate

By procedures essentially the same as those described in Example 8, the title compound was prepared. Mp 152-153°C.

### Example 19

### Preparation of 2-(4-Chlorophenyl)-1-phenyl-2-propylamine maleate

By procedures essentially the same as those described in Example 8, the title compound was prepared. Mp 171.5-173°C.

### Example 20

### Preparation of 2-(3,4-Dimethoxyphenyl)-1-phenyl-2-propylamine fumarate

By procedures essentially the same as those described in Example 8, the title compound was prepared. Mp 185°C (D).

### Example 21

### Preparation of 2-(4-methylphenyl)-1-phenyl-2-propylamine maleate

By procedures essentially the same as those described in Example 8, the title compound was prepared. Mp 169°C (D).

### Example 22

### Preparation of 2-(4-methoxyphenyl)-1-phenyl-2-propylamine maleate

By procedures essentially the same as those described in Example 8, the title compound was prepared. Mp 169-170°C.

### Example 23

### Preparation of 1-(3,4-dichlorophenyl)-2-phenyl-2-propylamine maleate

By procedures essentially the same as those described in Example 8, the title compound was prepared. Mp 175-176°C.

### Example 24

### Preparation of 1-(4-methoxyphenyl)-2-phenyl-2-propylamine maleate

By procedures essentially the same as those described in Example 8, the title compound was prepared. Mp 167-168°C.

### Example 25

### Preparation of 2-Amino-N-[1-(3-chlorophenyl)-2-phenyl-1-methylethyl]acetamide maleate

To a stirred solution of 2-(3-chlorophenyl)-1-phenyl-2-propylamine (9.0g, 0.037mol) in chloroform (100ml) under nitrogen was added N-(tert-butoxycarbonyl)glycine (7.0g, 0.04mol), and then a solution of dicyclohexylcarbodiimide (8.2g, 0.04mol) in chloroform (50ml) and the mixture stirred for 20 hours. The precipitated solid was removed by filtration and the solvent evaporated. The residue was dissolved in ethyl acetate (200ml) and filtered. The filtrate was washed with 1N HCl (100ml), 2N sodium carbonate (100ml) and saturated NaCl (100ml), dried, then evaporated to an oil, 14.2g. The above oil was dissolved in ethyl acetate (250ml), cooled in an ice water bath and the solution was acidified with gaseous HCl. The solution was warmed to ambient temperature and stirred overnight. The solvent was evaporated and the residue was partitioned between chloroform (200ml) and 3% NaOH (100ml). The chloroform solution was washed with saturated NaCl (100ml) and dried over magnesium sulphate. Removal of the solvent gave 10.7g of an oil. Treatment of this oil with maleic acid (4.1g, 0.035mol) in ethyl acetate (300ml) gave a white solid which was vacuum dried at 60°C for 60 hours to give 10.7g of the title compound, mp 160 - 161°C.

### Example 26

### Preparation of 2-Amino-N-[2-(2-chlorophenyl)-1-phenyl-1-methylethyl]acetamide maleate

By procedures essentially the same as those described in Example 25 the title compound was prepared. Mp 119 - 123°C.

### Example 27

### Preparation of 2-Amino-N-[2-(4-chlorophenyl)-1-phenyl-1-methylethyl]acetamide maleate

By procedures essentially the same as those described in Example 25 the title compound was prepared. Mp 162 - 164°C.

### Example 28

### Preparation of 2-Amino-N-[1-(2-methylphenyl)-2-phenyl-1-methylethyl]acetamide fumarate

By procedures essentially the same as those described in Example 25 the title compound was prepared. Mp 203°C (D).

### Example 29

### Preparation of 2-Amino-N-[1-(2-chlorophenyl)-2-phenyl-1-methylethyl]acetamide fumarate

By procedures essentially the same as those described in Example 25 the title compound was prepared. Mp 209°C (D).

### Example 30

### Preparation of 2-Amino-N-[2-(3-chlorophenyl)-1-phenyl-1-methylethyl]acetamide maleate

By procedures essentially the same as those described in Example 25 the title compound was prepared. Mp 171 - 172°C.

### Example 31

### Preparation of 2-Amino-N-[2-(3-aminophenyl)-1-phenyl-1-methylethyl]acetamide fumarate

By procedures essentially the same as those described in Example 25 the title compound was prepared. Mp 189 - 190°C.

### Example 32

### Preparation of 2-Amino-N-[2-(3-bromophenyl)-1-phenyl-1-methylethyl]acetamide maleate

By procedures essentially the same as those described in Example 25 the title compound was prepared. Mp 168 - 169°C.

### Example 33

### Preparation of 2-Amino-N-[2-(3-nitrophenyl)-1-phenyl-1-methylethyl]acetamide maleate

To a stirred solution of 1-(3-nitrophenyl)-2-phenyl-2-propylamine (10.0g, 0.039mol) in dichloromethane (250ml) under nitrogen was added N-phthaloylglycine (8.87g, 0.043mol), and then a solution of dicyclohexylcarbodiimide (8.4g, 0.041mol) in dichloromethane (75ml) and the mixture stirred for 20 hours. The precipitated solid was removed by filtration and the solid evaporated to give 22.3g of a white solid. This solid was slurried in hot ethanol (200ml) to provide 16.4g of 2-(1,3-dioxoisoindol-2-yl)-N-[2-(3-nitrophenyl)-1-phenyl-1-methylethyl]acetamide, mp 172-173°C. To a stirred suspension of the above compound (8.0g, 0.018mol) in absolute ethanol (150ml) was added 65% hydrazine hydrate (2.0ml, 0.054mol) and the mixture was heated to 40°C for 48 hours. The precipitated solid was removed by filtration and the solvent evaporated. The residue was dissolved in 3% NaOH (200ml) and extracted with chloroform (3 x 100ml). The combined chloroform extracts were dried over magnesium sulphate and the solvent evaporated to an oil 4.3g. The above oil was dissolved in ethyl acetate (200ml) and treated with maleic acid (1.7g, 0.015mol). The white solid which formed was isolated by filtration and vacuum dried at 50°C for 128 hours to provide 3.7g of the title compound, mp 170 - 171°C.

### Example 34

### Preparation of 2-Amino-N-[2-phenyl-1-(3-nitrophenyl)-1-methylethyl]acetamide maleate

By procedures essentially the same as those described in Example 33, the title compound was prepared. Mp 165 - 167°C.

### Example 35

### Preparation of 2-Amino-N-[1-(3-aminophenyl)-2-phenyl-1-methylethyl]acetamide maleate

To a stirred solution of 1-phenyl-2-[3-[(2,2,2-trichloroethoxycarbonyl)amino]phenyl]-2-propylamine (9.9g, 0.025mol) in chloroform (100ml) under nitrogen were added N-CBZ-glycine (5.7g, 0.027mol) and then a solution of dicyclohexylcarbodiimide (5.6g, 0.027mol) in chloroform (70ml) and the mixture was stirred for 18 hours. The precipitated solid was removed by filtration and the solvent evaporated. The residue was dissolved in ethyl acetate (200ml) and filtered. The filtrate was washed with 1N HCl (100ml), 2N sodium carbonate (100ml), saturated NaCl (100ml), dried and evaporated to an oil, 15.3g.

To a solution of the above oil in tetrahydrofuran (150ml) and 90% acetic acid (150ml) was added zinc dust (25g, 0.38mol) and the mixture stirred at ambient temperature for 20 hours. The reaction mixture was filtered, the filtrate dissolved in water (400ml) and ethyl acetate (300ml) and this mixture basified with solid sodium carbonate. The phases were separated and the aqueous phase was extracted with ethyl acetate (200ml). The combined organic extracts were washed with saturated NaCl (200ml) and dried over magnesium sulphate. Removal of solvents gave 10.3g of an oil. This oil was purified by silica gel chromatography on a Waters prep. HPLC, eluting with 50% ethyl acetate/hexane to provide 7.3g of an oil. This oil was dissolved in methanol (225ml) and 1N HCl (75ml), and hydrogenated at 276 kPa [40psi] in a Parr apparatus over 1.8g of 5% Pd/C catalyst for 2 hours. The catalyst was removed by filtration, and the solvent was evaporated. The residual oil was dissolved in 1N sodium carbonate (150ml) and extracted with chloroform (3 x 100ml). The combined extracts were washed with saturated NaCl (100ml), dried over magnesium sulphate, and the solvent evaporated to provide an oil 4.5g. This oil was dissolved in ethyl acetate (75ml) and treated with maleic acid (2.1g, 0.018mol). The white solid which formed was isolated by filtration and vacuum dried at 60°C for 48 hours to provide 5.15g of the title compound, mp 148 - 150°C.

### Example 36

### Preparation of 2-Amino-N-[1-(4-chlorophenyl)-2-phenyl-1-methylethyl]acetamide maleate

By procedures essentially the same as those described in Example 25, the title compound was prepared. Mp 172.5 - 175.5°C.

### Example 37

### Preparation of 2-Amino-N-[1-(3,4-dichlorophenyl)-2-phenyl-1-methylethyl]acetamide maleate

By procedures essentially the same as those described in Example 25, the title compound was prepared. Mp 186.5 - 187.5°C.

### Example 38

### Preparation of 2-Amino-N-[1-(3,4-dimethoxyphenyl)-2-phenyl-1-methylethyl]acetamide maleate

By procedures essentially the same as those described in Example 25, the title compound was prepared. Mp 174.5°C (D).

### Example 39

### Preparation of 2-Amino-N-[2-(3-cyanophenyl)-1-phenyl-1-methylethyl]acetamide maleate

By procedures essentially the same as those described in Example 25, the title compound was prepared. Mp 165 - 166°C.

### Example 40

### Preparation of 2-Amino-N-[1-(4-methylphenyl)-2-phenyl-1-methylethyl]acetamide maleate

By procedures essentially the same as those described in Example 25, the title compound was prepared. Mp 163°C.

### Example 41

### Preparation of 2-Amino-N-[2-phenyl-1-(4-hydroxyphenyl)-1-methylethyl]acetamide maleate

To a stirred solution of 1-phenyl-2-[4-(phenylmethoxy)phenyl]-2-propylamine (3.77g, 0.012mol) in dichloromethane (50ml) under nitrogen were added N-CBZ-glycine (2.47g, 0.012mol) and then a solution of dicyclohexylcarbodiimide (2.45g, 0.012mol) in dichloromethane (25ml), and the mixture stirred for 14 hours. The precipitated solid was removed by filtration and the solvent evaporated. The residue was dissolved in ethyl acetate (100ml) and filtered again. The ethyl acetate solution was washed with 1N HCl, 1N sodium hydroxide, saturated NaCl, dried over magnesium sulphate and the solvent evaporated to give 6.1g of an oil. This oil was dissolved in ethanol (150ml), tetrahydrofuran (25ml) and 1N HCl (50ml), and hydrogenated at 276 kPa [40psi] in a Parr apparatus over 5% Pd/C (0.8g) for 4 hours. The catalyst was removed by filtration and the solvents evaporated to give a solid. This solid was suspended in 1N sodium carbonate (100ml) and extracted with ethyl acetate (250ml) containing methanol (50ml). The organic solution was washed with saturated sodium chloride solution, dried over magnesium sulphate and the solvent evaporated to leave a white solid, 1.82g. This solid was dissolved in methanol (100ml) and maleic acid (0.74g, 0.0064mol) was added. The solvent was evaporated to an oil. This oil was crystallised from ethyl acetate-methanol and vacuum dried to provide 1.91g of the title compound, mp 174 - 175°C.

### Example 42

### Preparation of 2-Amino-N-[1-(4-hydroxyphenyl)-2-phenylethyl]acetamide maleate

By procedures essentially the same as those described in Example 41 the title compound was prepared. Mp 184-185°C.

### Example 43

### Preparation of 2-Amino-N-[2-(4-hydroxyphenyl)-1-phenyl-1-methylethyl]acetamide maleate

By procedures essentially the same as those described in Example 41 the title compound was prepared. Mp 167 - 168°C.

### Example 44

### Preparation of 2-Amino-N-[2-(4-hydroxyphenyl)-1-phenylethyl]acetamide acetate

To a stirred solution of 2-[4-(phenylmethoxy)phenyl]-1-phenylethylamine (6.51g, 0.022mol) in chloroform (100ml) under nitrogen were added N-CBZ-glycine (4.35g, 0.021mol) and then a solution of dicyclohexylcarbodiimide (4.42g, 0.021mol) in chloroform (50ml) and the mixture stirred for 24 hours. The precipitated solid was removed by filtration. The filter cake was suspended in warm tetrahydrofuran (150ml) and the insoluble material was removed by filtration. The filtrates were combined and the solvents evaporated to a white solid, 10.7g. This solid was recrystallised from ethyl acetate and cyclohexane to provide 8.5g of a white solid, mp 155 - 157°C. This solid was dissolved in tetrahydrofuran (300ml) and acetic acid (70ml) and hydrogenated at 276 kPa [40psi] in a Parr apparatus over 5% Pd/C (1.5g) for 17 hours. The catalyst was removed by filtration and the solvent evaporated to leave a white solid. This solid was recrystallised twice from ethyl acetate and methanol, and vacuum dried to provide 2.53g of the title compound, mp 145 - 147°C.

### Example 45

### Preparation of 2-Amino-N-[1,2-bis(4-hydroxyphenyl)-1-methyl-ethyl]acetamide acetate

By procedures essentially the same as those described in Example 44 the title compound was prepared. Mp 225 - 228°C (D).

### Example 46

### Preparation of 2-Amino-N-[1,2-bis(4-hydroxyphenyl)ethyl]acetamide acetate

By procedures essentially the same as those described in Example 44 the title compound was prepared. Mp 192 - 193°C.

### Example 47

### Preparation of 2-Amino-N-[1-(3-methoxyphenyl)-2-phenyl-1-methylethyl]acetamide maleate

By procedures essentially the same as those described in Example 44 the title compound was prepared. Mp 150°C.

### Example 48

### Preparation of 2-Amino-N-[1-(4-methoxyphenyl)-2-phenyl-1-methylethyl]acetamide maleate

By procedures essentially the same as those described in Example 33 the title compound was prepared. Mp 157-158°C.

### Example 49

### Preparation of 2-Amino-N-[2-(3,4-dichlorophenyl)-1-phenyl-1-methylethyl]acetamide maleate

By procedures essentially the same as those described in Example 25 the title compound was prepared. Mp 172.5 - 174°C.

### Example 50

### Preparation of 2-Amino-N-[2-(4-methoxyphenyl)-1-phenyl-1-methylethyl]acetamide maleate

By procedures essentially the same as those described in Example 33 the title compound was prepared. Mp 165 - 167°C.

## Claims (Claims for the following Contracting State(s): AT, BE, CH, DE, FR, GB, IT, LI, LU, NL, SE)

1. The use of a compound of formula I, 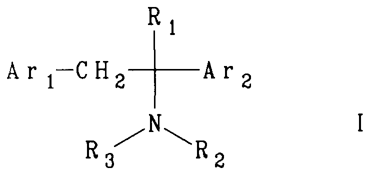 wherein
Ar₁ and Ar₂ independently represent phenyl substituted by one or more of amino, nitro, chlorine, bromine, hydroxy, C₁₋₆ alkoxy, C₁₋₆ alkyl or cyano; in addition one of Ar₁ or Ar₂ may represent phenyl;
R₁ represents hydrogen or C₁₋₆ alkyl;
R₂ represents hydrogen or COCH₂NH₂;
R₃ represents hydrogen or C₁₋₆ alkyl;
in addition, when R₂ represents hydrogen, then both of Ar₁ and Ar₂ may represent phenyl, one or both of Ar₁ and Ar₂ may represent fluorophenyl or 2-, 3- or 4-pyridinyl, and R₁ may represent C₁₋₆ alkoxycarbonyl or trifluoromethyl;
provided that:
(a) when R₁ and R₂ each represent H and Ar₁ and Ar₂ each represent phenyl, then R₃ is other than C₁₋₆ alkyl;
(b) when Ar₂ represents 2-, 3- or 4-pyridinyl, then R₁ represents C₁₋₆ alkyl; and
(c) when R₁ and R₂ each represent H, then Ar₁ does not represent phenyl substituted by amino;
or a pharmaceutically acceptable salt thereof;
as active ingredient in the manufacture of a neuroprotective or anticonvulsant medicament.

2. The use as claimed in claim 1, wherein the compound of formula I is:
1,2-diphenylethylamine;
1,2-diphenyl-2-propylamine;
1,2-bis(4-fluorophenyl)-2-propylamine;
1,2-diphenyl-2-butylamine;
(-)-1,2-diphenyl-2-propylamine;
(+)-1,2-diphenyl-2-propylamine;
2,3-diphenyl-2-aminopropanoic acid methyl ester;
N-methyl-1,2-diphenyl-2-propylamine;
1-(3-nitrophenyl)-2-phenyl-2-propylamine;
1-(3-chlorophenyl)-2-phenyl-2-propylamine;
1-(3-bromophenyl)-2-phenyl-2-propylamine;
1-(3-cyanophenyl)-2-phenyl-2-propylamine;
2-(2-methylphenyl)-1-phenyl-2-propylamine;
1-(4-chlorophenyl)-2-phenyl-2-propylamine;
1-phenyl-2-(3,4-dichlorophenyl)-2-propylamine;
1-phenyl-2-(3-methoxyphenyl)-2-propylamine;
1-(4-hydroxyphenyl)-2-phenyl-2-propylamine;
1-(4-hydroxyphenyl)-2-phenylethylamine;
1-phenyl-2-(4-hydroxyphenyl)ethylamine;
1,2-bis(4-hydroxyphenyl)ethylamine;
1-phenyl-2-(4-hydroxyphenyl)-2-propylamine;
1,2-bis(4-hydroxyphenyl)-2-propylamine;
1-phenyl-2-(2-pyridinyl)ethylamine;
1-phenyl-2-(3-pyridinyl)ethylamine;
1-phenyl-2-(4-pyridinyl)ethylamine;
3,3,3-trifluoro-1,2-diphenyl-2-propylamine;
N-methyl-3,3,3-trifluoro-1,2-diphenyl-2-propylamine;
or a pharmaceutically acceptable salt thereof.

3. 1-Phenyl-2-(2-pyridinyl)ethylamine, or a pharmaceutically acceptable salt thereof, for use as a pharmaceutical.

4. A pharmaceutical composition comprising 1-phenyl-2-(2-pyridinyl)ethylamine, or a pharmaceutically acceptable salt thereof, in admixture with a pharmaceutically acceptable adjuvant, diluent or carrier.

5. A compound of formula IA, 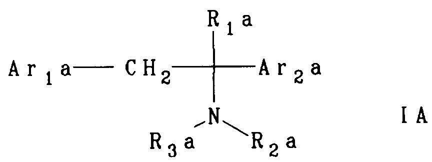 wherein
Ar₁a and Ar₂a independently represent phenyl substituted by one or more of amino, nitro, chlorine, bromine, hydroxy, C₁₋₆ alkoxy, C₁₋₆ alkyl or cyano; in addition one of Ar₁a or Ar₂a may represent phenyl;
R₁a represents hydrogen or C₁₋₆ alkyl;
R₂a represents hydrogen or COCH₂NH₂;
R₃a represents hydrogen or C₁₋₆ alkyl;
provided that when R₂a represents hydrogen, then R₁a represents C₁₋₆ alkyl;
or a pharmaceutically acceptable salt thereof.

6. A compound as claimed in claim 5, wherein one or both of Ar₁a and Ar₂a represents 4-hydroxyphenyl.

7. A compound as claimed in claim 5, wherein Ar₁a and Ar₂a are mono- or disubstituted.

8. A compound as claimed in claim 5, wherein R₁a represents methyl.

9. A compound as claimed in claim 5, wherein R₃a represents hydrogen.

10. A compound as claimed in claim 5, which is:
1-(4-hydroxyphenyl)-2-phenyl-2-propylamine;
1-phenyl-2-(4-hydroxyphenyl)-2-propylamine;
1,2-bis(4-hydroxyphenyl)-2-propylamine;
1-(3-nitrophenyl)-2-phenyl-2-propylamine;
1-(3-chlorophenyl)-2-phenyl-2-propylamine;
1-(3-bromophenyl)-2-phenyl-2-propylamine;
1-(3-cyanophenyl)-2-phenyl-2-propylamine;
2-(2-methylphenyl)-1-phenyl-2-propylamine;
1-(4-chlorophenyl)-2-phenyl-2-propylamine;
1-phenyl-2-(3,4-dichlorophenyl)-2-propylamine;
1-phenyl-2-(3-methoxyphenyl)-2-propylamine;
2-(3-chlorophenyl)-1-phenyl-2-propylamine;
1-(2-chlorophenyl)-2-phenyl-2-propylamine;
2-(2-chlorophenyl)-1-phenyl-2-propylamine;
2-(3-nitrophenyl)-1-phenyl-2-propylamine;
2-(4-chlorophenyl)-1-phenyl-2-propylamine;
2-(3,4-dimethoxyphenyl)-1-phenyl-2-propylamine;
2-(4-methylphenyl)-1-phenyl-2-propylamine;
2-(4-methoxyphenyl)-1-phenyl-2-propylamine;
1-(3,4-dichlorophenyl)-2-phenyl-2-propylamine;
1-(4-methoxyphenyl)-2-phenyl-2-propylamine;
2-amino-N-[1-(3-chlorophenyl)-2-phenyl-1-methylethyl]acetamide;
2-amino-N-[2-(2-chlorophenyl)-1-phenyl-1-methylethyl]acetamide;
2-amino-N-[2-(4-chlorophenyl)-1-phenyl-1-methylethyl]acetamide;
2-amino-N-[1-(2-methylphenyl)-2-phenyl-1-methylethyl]acetamide;
2-amino-N-[1-(2-chlorophenyl)-2-phenyl-1-methylethyl]acetamide;
2-amino-N-[2-(3-chlorophenyl)-1-phenyl-1-methylethyl]acetamide;
2-amino-N-[2-(3-aminophenyl)-1-phenyl-1-methylethyl]acetamide;
2-amino-N-[2-(3-bromophenyl)-1-phenyl-1-methylethyl]acetamide;
2-amino-N-[2-(3-nitrophenyl)-1-phenyl-1-methylethyl]acetamide;
2-amino-N-[1-(3-nitrophenyl)-2-phenyl-1-methylethyl]acetamide;
2-amino-N-[1-(3-aminophenyl)-2-phenyl-1-methylethyl]acetamide;
2-amino-N-[1-(4-chlorophenyl)-2-phenyl-1-methylethyl]acetamide;
2-amino-N-[1-(3,4-dichlorophenyl)-2-phenyl-1-methylethyl]acetamide;
2-amino-N-[1-(3,4-dimethoxyphenyl)-2-phenyl-1-methylethyl]acetamide;
2-amino-N-[2-(3-cyanophenyl)-1-phenyl-1-methylethyl]acetamide;
2-amino-N-[1-(4-methylphenyl)-2-phenyl-1-methylethyl]acetamide;
2-amino-N-[1-(4-hydroxyphenyl)-2-phenyl-1-methylethyl]acetamide;
2-amino-N-[1-(4-hydroxyphenyl)-2-phenylethyl]acetamide;
2-amino-N-[2-(4-hydroxyphenyl)-1-phenyl-1-methylethyl]acetamide;
2-amino-N-[2-(4-hydroxyphenyl)-1-phenylethyl]acetamide;
2-amino-N-[1,2-bis(4-hydroxyphenyl)-1-methylethyl]-acetamide;
2-amino-N-[1,2-bis(4-hydroxyphenyl)ethyl]acetamide;
2-amino-N-[1-(3-methoxyphenyl)-2-phenyl-1-methylethyl]-acetamide;
2-amino-N-[1-(4-methoxyphenyl)-2-phenyl-1-methylethyl]-acetamide;
2-amino-N-[2-(3,4-dichlorophenyl)-1-phenyl-1-methyl-ethyl]acetamide;
2-amino-N-[2-(4-methoxyphenyl)-1-phenyl-1-methyl-ethyl]acetamide;
or a pharmaceutically acceptable salt thereof.

11. A compound of formula IA, as defined in claim 5, or a pharmaceutically acceptable salt thereof, for use as a pharmaceutical.

12. A pharmaceutical composition comprising a compound of formula IA, as defined in claim 5, or a pharmaceutically acceptable salt thereof, in admixture with a pharmaceutically acceptable adjuvant, diluent or carrier.

13. A process for the preparation of a compound of formula IA, as defined in claim 5, or a pharmaceutically acceptable salt thereof, which comprises:
a) for compounds in which R₂a represents hydrogen and R₃a represents C₁₋₆ alkyl, alkylation of a corresponding compound of formula IA in which R₃a represents hydrogen;
b) for compounds in which R₂a and R₃a both represent hydrogen, amide hydrolysis of a corresponding compound of formula IIA, 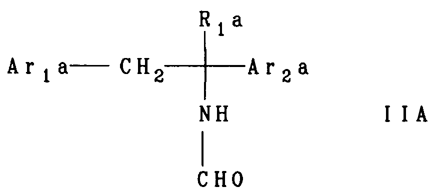 in which Ar₁a, Ar₂a and R₁a are as defined in claim 5;
c) for compounds in which R₂a and R₃a both represent hydrogen, reductive carbamate cleavage of a compound of formula IIIA, 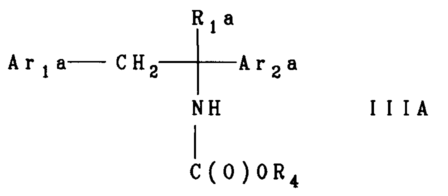 in which Ar₁a, Ar₂a and R₁a are as defined in claim 5 and R₄ represents an alkyl or aryl group;
d) for compounds in which R₂a represents COCH₂NH₂, removal of the amine protecting group from a compound of formula IVA, 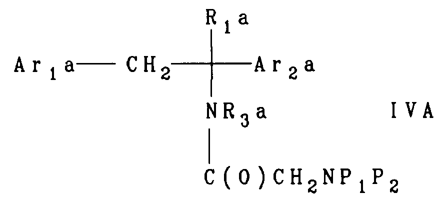 in which P₁ and P₂ together constitute a suitable protecting group, and Ar₁a, Ar₂a, R₁a and R₃a are as defined in claim 5; or
e) for compounds in which R₂a represents COCH₂NH₂, aminating a compound of formula VA, 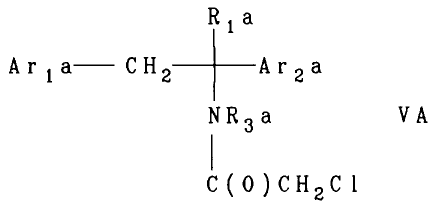 in which Ar₁a, Ar₂a, R₁a and R₃a are as defined in claim 5.

## Claims (Claims for the following Contracting State(s): ES, GR)

1. A process for the production of a pharmaceutical composition comprising 1-phenyl-2-(2-pyridinyl)ethylamine, or a pharmaceutically acceptable salt thereof, in admixture with a pharmaceutically acceptable adjuvant, diluent or carrier; which comprises mixing the ingredients.

2. A process for the production of a compound of formula IA, 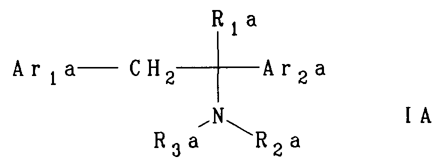 wherein
Ar₁a and Ar₂a independently represent phenyl substituted by one or more of amino, nitro, chlorine, bromine, hydroxy, C₁₋₆ alkoxy, C₁₋₆ alkyl or cyano; in addition one of Ar₁a or Ar₂a may represent phenyl;
R₁a represents hydrogen or C₁₋₆ alkyl;
R₂a represents hydrogen or COCH₂NH₂;
R₃a represents hydrogen or C₁₋₆ alkyl;
provided that when R₂a represents hydrogen, then R₁a represents C₁₋₆ alkyl;
or a pharmaceutically acceptable salt thereof;
which comprises:
a) for compounds in which R₂a represents hydrogen and R₃a represents C₁₋₆ alkyl, alkylation of a corresponding compound of formula IA in which R₃a represents hydrogen;
b) for compounds in which R₂a and R₃a both represent hydrogen, amide hydrolysis of a corresponding compound of formula IIA, 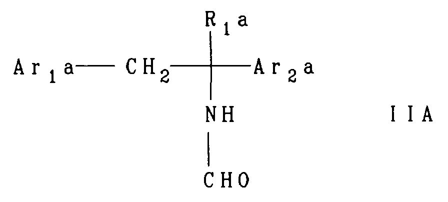 in which Ar₁a, Ar₂a and R₁a are as defined above;
c) for compounds in which R₂a and R₃a both represent hydrogen, reductive carbamate cleavage of a compound of formula IIIA, 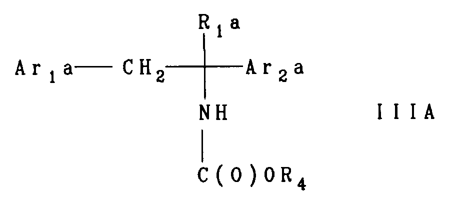 in which Ar₁a, Ar₂a and R₁a are as defined above and R₄ represents an alkyl or aryl group;
d) for compounds in which R₂a represents COCH₂NH₂, removal of the amine protecting group from a compound of formula IVA, 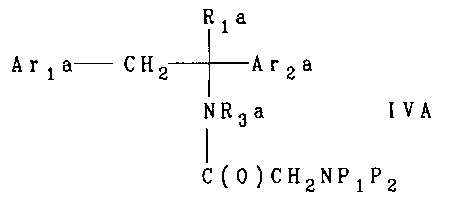 in which P₁ and P₂ together constitute a suitable protecting group, and Ar₁a, Ar₂a, R₁a and R₃a are as defined above; or
e) for compounds in which R₂a represents COCH₂NH₂, aminating a compound of formula VA, 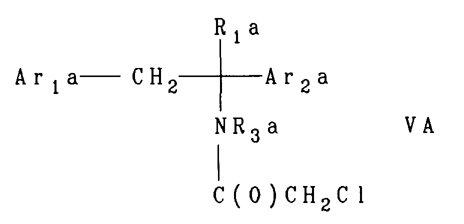 in which Ar₁a, Ar₂a, R₁a and R₃a are as defined above.

3. The process as claimed in claim 2, wherein one or both of Ar₁a and Ar₂a represents 4-hydroxyphenyl.

4. The process as claimed in claim 2, wherein Ar₁a and Ar₂a are mono- or disubstituted.

5. The process as claimed in claim 2, wherein R₁a represents methyl.

6. The process as claimed in claim 2, wherein R₃a represents hydrogen.

7. The process as claimed in claim 2, wherein the compound of formula IA is:
1-(4-hydroxyphenyl)-2-phenyl-2-propylamine;
1-phenyl-2-(4-hydroxyphenyl)-2-propylamine;
1,2-bis(4-hydroxyphenyl)-2-propylamine;
1-(3-nitrophenyl)-2-phenyl-2-propylamine;
1-(3-chlorophenyl)-2-phenyl-2-propylamine;
1-(3-bromophenyl)-2-phenyl-2-propylamine;
1-(3-cyanophenyl)-2-phenyl-2-propylamine;
2-(2-methylphenyl)-1-phenyl-2-propylamine;
1-(4-chlorophenyl)-2-phenyl-2-propylamine;
1-phenyl-2-(3,4-dichlorophenyl)-2-propylamine;
1-phenyl-2-(3-methoxyphenyl)-2-propylamine;
2-(3-chlorophenyl)-1-phenyl-2-propylamine;
1-(2-chlorophenyl)-2-phenyl-2-propylamine;
2-(2-chlorophenyl)-1-phenyl-2-propylamine;
2-(3-nitrophenyl)-1-phenyl-2-propylamine;
2-(4-chlorophenyl)-1-phenyl-2-propylamine;
2-(3,4-dimethoxyphenyl)-1-phenyl-2-propylamine;
2-(4-methylphenyl)-1-phenyl-2-propylamine;
2-(4-methoxyphenyl)-1-phenyl-2-propylamine;
1-(3,4-dichlorophenyl)-2-phenyl-2-propylamine;
1-(4-methoxyphenyl)-2-phenyl-2-propylamine;
2-amino-N-[1-(3-chlorophenyl)-2-phenyl-1-methylethyl]acetamide;
2-amino-N-[2-(2-chlorophenyl)-1-phenyl-1-methylethyl]acetamide;
2-amino-N-[2-(4-chlorophenyl)-1-phenyl-1-methylethyl]acetamide;
2-amino-N-[1-(2-methylphenyl)-2-phenyl-1-methylethyl]acetamide;
2-amino-N-[1-(2-chlorophenyl)-2-phenyl-1-methylethyl]acetamide;
2-amino-N-[2-(3-chlorophenyl)-1-phenyl-1-methylethyl]acetamide;
2-amino-N-[2-(3-aminophenyl)-1-phenyl-1-methylethyl]acetamide;
2-amino-N-[2-(3-bromophenyl)-1-phenyl-1-methylethyl]acetamide;
2-amino-N-[2-(3-nitrophenyl)-1-phenyl-1-methylethyl]acetamide;
2-amino-N-[1-(3-nitrophenyl)-2-phenyl-1-methylethyl]acetamide;
2-amino-N-[1-(3-aminophenyl)-2-phenyl-1-methylethyl]acetamide;
2-amino-N-[1-(4-chlorophenyl)-2-phenyl-1-methylethyl]acetamide;
2-amino-N-[1-(3,4-dichlorophenyl)-2-phenyl-1-methylethyl]acetamide;
2-amino-N-[1-(3,4-dimethoxyphenyl)-2-phenyl-1-methylethyl]acetamide;
2-amino-N-[2-(3-cyanophenyl)-1-phenyl-1-methylethyl]acetamide;
2-amino-N-[1-(4-methylphenyl)-2-phenyl-1-methylethyl]acetamide;
2-amino-N-[1-(4-hydroxyphenyl)-2-phenyl-1-methylethyl]acetamide;
2-amino-N-[1-(4-hydroxyphenyl)-2-phenylethyl]acetamide;
2-amino-N-[2-(4-hydroxyphenyl)-1-phenyl-1-methylethyl]acetamide;
2-amino-N-[2-(4-hydroxyphenyl)-1-phenylethyl]acetamide;
2-amino-N-[1,2-bis(4-hydroxyphenyl)-1-methylethyl]-acetamide;
2-amino-N-[1,2-bis(4-hydroxyphenyl)ethyl]acetamide;
2-amino-N-[1-(3-methoxyphenyl)-2-phenyl-1-methylethyl]-acetamide;
2-amino-N-[1-(4-methoxyphenyl)-2-phenyl-1-methylethyl]-acetamide;
2-amino-N-[2-(3,4-dichlorophenyl)-1-phenyl-1-methyl-ethyl]acetamide;
2-amino-N-[2-(4-methoxyphenyl)-1-phenyl-1-methyl-ethyl]acetamide;
or a pharmaceutically acceptable salt thereof.

8. A process for the production of a pharmaceutical composition comprising a compound of formula IA as defined in claim 2, or a pharmaceutically acceptable salt thereof, in admixture with a pharmaceutically acceptable adjuvant, diluent or carrier; which comprises mixing the ingredients.

## Patentansprüche (Patentansprüche für folgende(n) Vertragsstaat(en): AT, BE, CH, DE, FR, GB, IT, LI, LU, NL, SE)

1. Verwendung einer Verbindung der Formel (I) 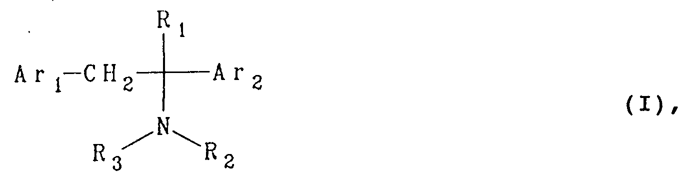 worin Ar₁ und Ar₂ unabhängig Phenyl, substituiert durch eines oder mehrere von Amino, Nitro, Chlor, Brom, Hydroxy, C₁-C₆-Alkoxy, C₁-C₆-Alkyl oder Cyano, bedeuten; zusätzlich eines von Ar₁ oder Ar₂ Phenyl darstellen kann; R₁ Wasserstoff oder C₁-C₆-Alkyl ist; R₂ Wasserstoff oder COCH₂NH₂ bedeutet; R₃ Wasserstoff oder C₁-C₆-Alkyl darstellt; zusätzlich, wenn R₂ Wasserstoff bedeutet, beide von Ar₁ und Ar₂ Phenyl darstellen können, eines oder beide von Ar₁ und Ar₂ Fluorphenyl oder 2-, 3- oder 4-Pyridinyl sein können, und R₁ die Bedeutung C₁-C₆-Alkoxycarbonyl oder Trifluormethyl haben kann; mit der Maßgabe, daß:
(a) wenn R₁ und R₂ jeweils H darstellen, und Ar₁ und Ar₂ jeweils Phenyl sind, R₃ von C₁-C₆-Alkyl verschieden ist;
(b) wenn Ar₂ 2-, 3- oder 4-Pyridinyl bedeutet, R₁ C₁-C₆-Alkyl darstellt; und
(c) wenn R₁ und R₂ jeweils H sind, Ar₁ nicht Phenyl, substituiert durch Amino, bedeutet;
oder eines pharmazeutisch annehmbaren Salzes hievon, als aktiver Bestandteil bei der Herstellung eines Neuro-Schutz- oder antikonvulsiven Medikaments.

2. Verwendung nach Anspruch 1, wobei die Verbindung der Formel (I) ist:
1,2-Diphenylethylamin;
1,2-Diphenyl-2-propylamin;
1,2-Bis-(4-fluorphenyl)-2-propylamin;
1,2-Diphenyl-2-butylamin;
(-)-1,2-Diphenyl-2-propylamin;
(+)-1,2-Diphenyl-2-propylamin;
2,3-Diphenyl-2-aminopropansäuremethylester;
N-Methyl-1,2-diphenyl-2-propylamin;
1-(3-Nitrophenyl)-2-phenyl-2-propylamin;
1-(3-Chlorphenyl)-2-phenyl-2-propylamin;
1-(3-Bromphenyl)-2-phenyl-2-propylamin;
1-(3-Cyanophenyl)-2-phenyl-2-propylamin;
2-(2-Methylphenyl)-1-phenyl-2-propylamin;
1-(4-Chlorphenyl)-2-phenyl-2-propylamin;
1-Phenyl-2-(3,4-dichlorphenyl)-2-propylamin;
1-Phenyl-2-(3-methoxyphenyl)-2-propylamin;
1-(4-Hydroxyphenyl)-2-phenyl-2-propylamin;
1-(4-Hydroxyphenyl)-2-phenylethylamin;
1-Phenyl-2-(4-hydroxyphenyl)-ethylamin;
1,2-Bis-(4-hydroxyphenyl)-ethylamin;
1-Phenyl-2-(4-hydroxyphenyl)-2-propylamin;
1,2-Bis-(4-hydroxyphenyl)-2-propylamin;
1-Phenyl-2-(2-pyridinyl)-ethylamin;
1-Phenyl-2-(3-pyridinyl)-ethylamin;
1-Phenyl-2-(4-pyridinyl)-ethylamin;
3,3,3-Trifluor-1,2-diphenyl-2-propylamin;
N-Methyl-3,3,3-trifluor-1,2-diphenyl-2-propylamin;
oder ein pharmazeutisch annehmbares Salz hievon.

3. 1-Phenyl-2-(2-pyridinyl)-ethylamin, oder ein pharmazeutisch annehmbares Salz hievon, zur Verwendung als Pharmazeutikum.

4. Pharmazeutische Zusammensetzung, welche 1-Phenyl-2-(2-pyridinyl)-ethylamin, oder ein pharmazeutisch annehmbares Salz hievon, in Mischung mit einem pharmazeutisch annehmbaren Adjuvans, Verdünnungsmittel oder Träger umfaßt.

5. Verbindung der Formel (IA) 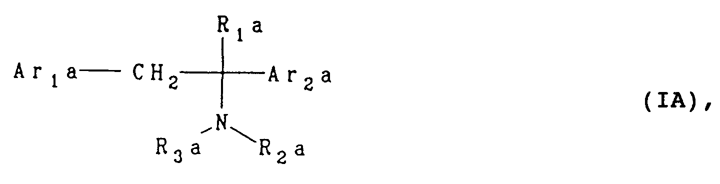 worin Ar₁a und Ar₂a unabhängig Phenyl, substituiert durch eines oder mehrere von Amino, Nitro, Chlor, Brom, Hydroxy, C₁-C₆-Alkoxy, C₁-C₆-Alkyl oder Cyano, bedeuten; zusätzlich eines von Ar₁a oder Ar₂a Phenyl darstellen kann; R₁a Wasserstoff oder C₁-C₆-Alkyl ist; R₂a Wasserstoff oder COCH₂NH₂ bedeutet; R₃a Wasserstoff oder C₁-C₆-Alkyl darstellt; mit der Maßgabe, daß, wenn R₂a Wasserstoff ist, R₁a C₁-C₆-Alkyl bedeutet; oder ein pharmazeutisch annehmbares Salz hievon.

6. Verbindung nach Anspruch 5, worin eines oder beide von Ar₁a und Ar₂a 4-Hydroxyphenyl darstellen.

7. Verbindung nach Anspruch 5, worin Ar₁a und Ar₂a mono- oder disubstituiert sind.

8. Verbindung nach Anspruch 5, worin R₁a Methyl ist.

9. Verbindung nach Anspruch 5, worin R₃a Wasserstoff bedeutet.

10. Verbindung nach Anspruch 5, welche ist:
1-(4-Hydroxyphenyl)-2-phenyl-2-propylamin;
1-Phenyl-2-(4-hydroxyphenyl)-2-propylamin;
1,2-Bis-(4-hydroxyphenyl)-2-propylamin;
1-(3-Nitrophenyl)-2-phenyl-2-propylamin;
1-(3-Chlorphenyl)-2-phenyl-2-propylamin;
1-(3-Bromphenyl)-2-phenyl-2-propylamin;
1-(3-Cyanophenyl)-2-phenyl-2-propylamin;
2-(2-Methylphenyl)-1-phenyl-2-propylamin;
1-(4-Chlorphenyl)-2-phenyl-2-propylamin;
1-Phenyl-2-(3,4-dichlorphenyl)-2-propylamin;
1-Phenyl-2-(3-methoxyphenyl)-2-propylamin;
2-(3-Chlorphenyl)-1-phenyl-2-propylamin;
1-(2-Chlorphenyl)-2-phenyl-2-propylamin;
2-(2-Chlorphenyl)-1-phenyl-2-propylamin;
2-(3-Nitrophenyl)-1-phenyl-2-propylamin;
2-(4-Chlorphenyl)-1-phenyl-2-propylamin;
2-(3,4-Dimethoxyphenyl)-1-phenyl-2-propylamin;
2-(4-Methylphenyl)-1-phenyl-2-propylamin;
2-(4-Methoxyphenyl)-1-phenyl-2-propylamin;
1-(3,4-Dichlorphenyl)-2-phenyl-2-propylamin;
1-(4-Methoxyphenyl)-2-phenyl-2-propylamin;
2-Amino-N-[1-(3-chlorphenyl)-2-phenyl-1-methylethyl]-acetamid;
2-Amino-N-[2-(2-chlorphenyl)-1-phenyl-1-methylethyl]-acetamid;
2-Amino-N-[2-(4-chlorphenyl)-1-phenyl-1-methylethyl]-acetamid;
2-Amino-N-[1-(2-methylphenyl)-2-phenyl-1-methylethyl]-acetamid;
2-Amino-N-[1-(2-chlorphenyl)-2-phenyl-1-methylethyl]-acetamid;
2-Amino-N-[2-(3-chlorphenyl)-1-phenyl-1-methylethyl]-acetamid;
2-Amino-N-[2-(3-aminophenyl)-1-phenyl-1-methylethyl]-acetamid;
2-Amino-N-[2-(3-bromphenyl)-1-phenyl-1-methylethyl]-acetamid;
2-Amino-N-[2-(3-nitrophenyl)-1-phenyl-1-methylethyl]-acetamid;
2-Amino-N-[1-(3-nitrophenyl)-2-phenyl-1-methylethyl]-acetamid;
2-Amino-N-[1-(3-aminophenyl)-2-phenyl-1-methylethyl]-acetamid;
2-Amino-N-[1-(4-chlorphenyl)-2-phenyl-1-methylethyl]-acetamid;
2-Amino-N-[1-(3,4-dichlorphenyl)-2-phenyl-1-methylethyl]-acetamid;
2-Amino-N-[1-(3,4-dimethoxyphenyl)-2-phenyl-1-methylethyl]-acetamid;
2-Amino-N-[2-(3-cyanophenyl)-1-phenyl-1-methylethyl]-acetamid;
2-Amino-N-[1-(4-methylphenyl)-2-phenyl-1-methylethyl]-acetamid;
2-Amino-N-[1-(4-hydroxyphenyl)-2-phenyl-1-methylethyl]-acetamid;
2-Amino-N-[1-(4-hydroxyphenyl)-2-phenylethyl]-acetamid;
2-Amino-N-[2-(4-hydroxyphenyl)-1-phenyl-1-methylethyl]-acetamid;
2-Amino-N-[2-(4-hydroxyphenyl)-1-phenylethyl]-acetamid;
2-Amino-N-[1,2-bis-(4-hydroxyphenyl)-1-methylethyl]-acetamid;
2-Amino-N-[1,2-bis-(4-hydroxyphenyl)-ethyl]-acetamid;
2-Amino-N-[1-(3-methoxyphenyl)-2-phenyl-1-methylethyl]-acetamid;
2-Amino-N-[1-(4-methoxyphenyl)-2-phenyl-1-methylethyl]-acetamid;
2-Amino-N-[2-(3,4-dichlorphenyl)-1-phenyl-1-methylethyl]-acetamid;
2-Amino-N-[2-(4-methoxyphenyl)-1-phenyl-1-methylethyl]-acetamid;
oder ein pharmazeutisch annehmbares Salz hievon.

11. Verbindung der Formel (IA), wie in Anspruch 5 definiert, oder ein pharmazeutisch annehmbares Salz hievon, zur Verwendung als Pharmazeutikum.

12. Pharmazeutische Zusammensetzung, welche eine Verbindung der Formel (IA), wie in Anspruch 5 definiert, oder ein pharmazeutisch annehmbares Salz hievon, in Mischung mit einem pharmazeutisch annehmbaren Adjuvans, Verdünnungsmittel oder Träger umfaßt.

13. Verfahren zur Herstellung einer Verbindung der Formel (IA), wie in Anspruch 5 definiert, oder eines pharmazeutisch annehmbaren Salzes hievon, welches Verfahren umfaßt:
a) für Verbindungen, worin R₂a Wasserstoff bedeutet, und R₃a C₁-C₆-Alkyl darstellt, Alkylierung einer entsprechenden Verbindung der Formel (IA), worin R₃a Wasserstoff ist;
b) für Verbindungen, worin R₂a und R₃a beide Wasserstoff bedeuten, Amidhydrolyse einer entsprechenden Verbindung der Formel (IIA) 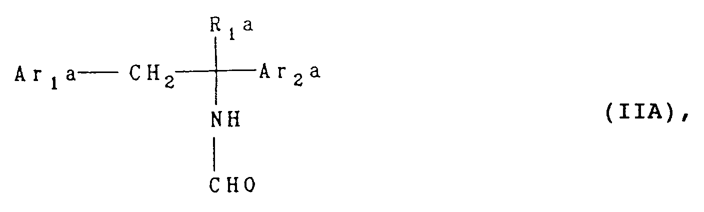 worin Ar₁a, Ar₂a und R₁a wie in Anspruch 5 definiert sind;
c) für Verbindungen, worin R₂a und R₃a beide Wasserstoff bedeuten, reduktive Carbamatspaltung einer Verbindung der Formel (IIIA) 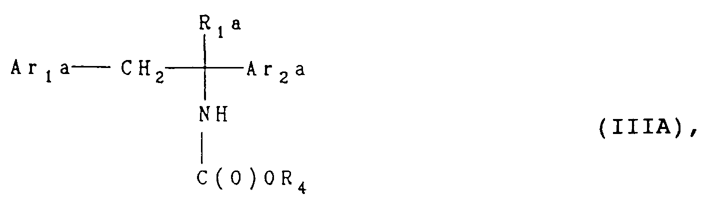 worin Ar₁a, Ar₂a und R₁a wie in Anspruch 5 definiert sind, und R₄ eine Alkyl- oder Aryl-Gruppe darstellt;
d) für Verbindungen, worin R₂a die Bedeutung COCH₂NH₂ hat, Entfernung der Amin-Schutzgruppe aus einer Verbindung der Formel (IVA) 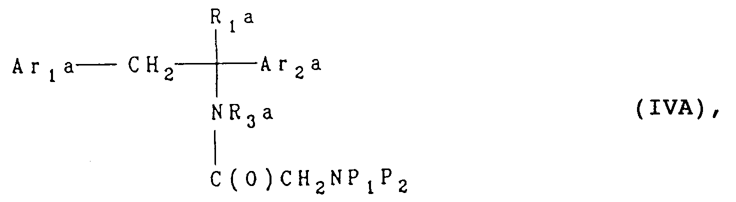 worin P₁ und P₂ zusammen eine geeignete Schutzgruppe bilden, und Ar₁a, Ar₂a, R₁a und R₃a wie in Anspruch 5 definiert sind; oder
e) für Verbindungen, worin R₂a die Bedeutung COCH₂NH₂ hat, Aminierung einer Verbindung der Formel (VA) 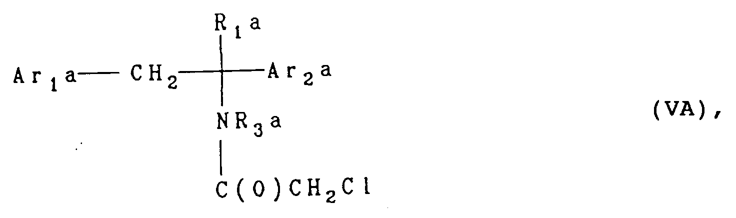 worin Ar₁a, Ar₂a, R₁a und R₃a wie in Anspruch 5 definiert sind.

## Patentansprüche (Patentansprüche für folgende(n) Vertragsstaat(en): ES, GR)

1. Verfahren zur Herstellung einer pharmazeutischen Zusammensetzung, die 1-Phenyl-2-(2-pyridinyl)-ethylamin, oder ein pharmazeutisch annehmbares Salz hievon, in Mischung mit einem pharmazeutisch annehmbaren Adjuvans, Verdünnungsmittel oder Träger umfaßt, welches Verfahren das Mischen der Bestandteile umfaßt.

2. Verfahren zur Herstellung einer Verbindung der Formel (IA) 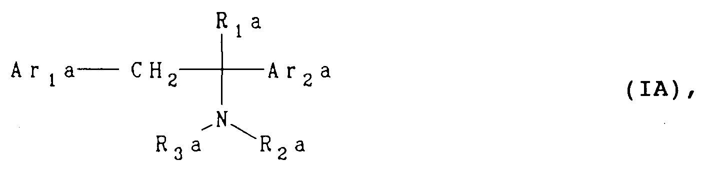 worin Ar₁a und Ar₂a unabhängig Phenyl, substituiert durch eines oder mehrere von Amino, Nitro, Chlor, Brom, Hydroxy, C₁-C₆-Alkoxy, C₁-C₆-Alkyl oder Cyano, bedeuten; zusätzlich eines von Ar₁a oder Ar₂a Phenyl darstellen kann; R₁a Wasserstoff oder C₁-C₆-Alkyl ist; R₂a Wasserstoff oder COCH₂NH₂ bedeutet; R₃a Wasserstoff oder C₁-C₆-Alkyl darstellt; mit der Maßgabe, daß, wenn R₂a Wasserstoff ist, R₁a C₁-C₆-Alkyl bedeutet;
oder eines pharmazeutisch annehmbaren Salzes hievon, welches Verfahren umfaßt:
a) für Verbindungen, worin R₂a Wasserstoff bedeutet, und R₃a C₁-C₆-Alkyl darstellt, Alkylierung einer entsprechenden Verbindung der Formel (IA), worin R₃a Wasserstoff ist;
b) für Verbindungen, worin R₂a und R₃a beide Wasserstoff bedeuten, Amidhydrolyse einer entsprechenden Verbindung der Formel (IIA) 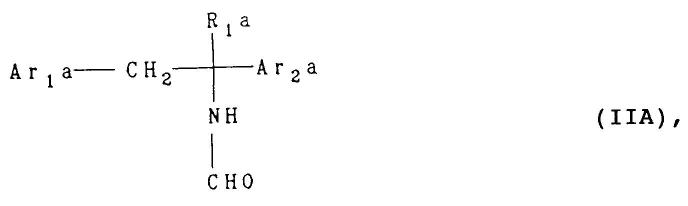 worin Ar₁a, Ar₂a und R₁a wie oben definiert sind;
c) für Verbindungen, worin R₂a und R₃a beide Wasserstoff bedeuten, reduktive Carbamatspaltung einer Verbindung der Formel (IIIA) 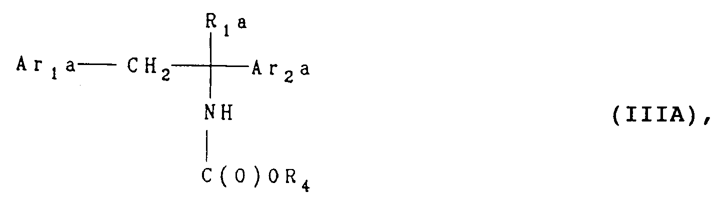 worin Ar₁a, Ar₂a und R₁a wie oben definiert sind, und R₄ eine Alkyl- oder Aryl-Gruppe darstellt;
d) für Verbindungen, worin R₂a die Bedeutung COCH₂NH₂ hat, Entfernung der Amin-Schutzgruppe aus einer Verbindung der Formel (IVA) 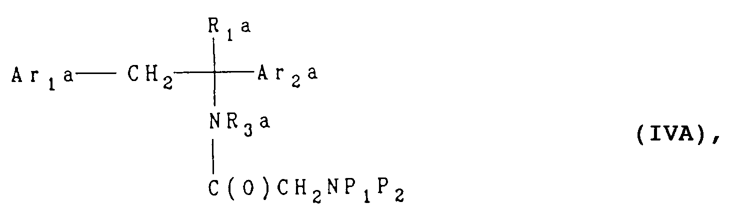 worin P₁ und P₂ zusammen eine geeignete Schutzgruppe bilden, und Ar₁a, Ar₂a, R₁a und R₃a wie oben definiert sind; oder
e) für Verbindungen, worin R₂a die Bedeutung COCH₂NH₂ hat, Aminierung einer Verbindung der Formel (VA) 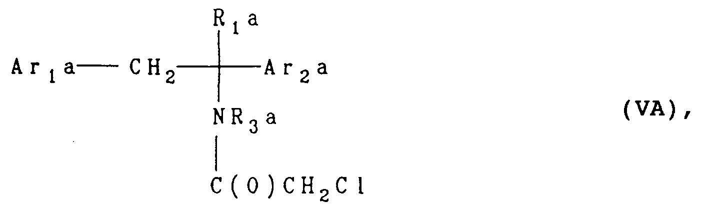 worin Ar₁a, Ar₂a, R₁a und R₃a wie oben definiert sind.

3. Verfahren nach Anspruch 2, wobei eines oder beide von Ar₁a und Ar₂a 4-Hydroxyphenyl darstellen.

4. Verfahren nach Anspruch 2, wobei Ar₁a und Ar₂a mono- oder disubstituiert sind.

5. Verfahren nach Anspruch 2, wobei R₁a Methyl ist.

6. Verfahren nach Anspruch 2, wobei R₃a Wasserstoff bedeutet.

7. Verfahren nach Anspruch 2, bei welchem die Verbindung der Formel (IA) ist:
1-(4-Hydroxyphenyl)-2-phenyl-2-propylamin;
1-Phenyl-2-(4-hydroxyphenyl)-2-propylamin;
1,2-Bis-(4-hydroxyphenyl)-2-propylamin;
1-(3-Nitrophenyl)-2-phenyl-2-propylamin;
1-(3-Chlorphenyl)-2-phenyl-2-propylamin;
1-(3-Bromphenyl)-2-phenyl-2-propylamin;
1-(3-Cyanophenyl)-2-phenyl-2-propylamin;
2-(2-Methylphenyl)-1-phenyl-2-propylamin;
1-(4-Chlorphenyl)-2-phenyl-2-propylamin;
1-Phenyl-2-(3,4-dichlorphenyl)-2-propylamin;
1-Phenyl-2-(3-methoxyphenyl)-2-propylamin;
2-(3-Chlorphenyl)-1-phenyl-2-propylamin;
1-(2-Chlorphenyl)-2-phenyl-2-propylamin;
2-(2-Chlorphenyl)-1-phenyl-2-propylamin;
2-(3-Nitrophenyl)-1-phenyl-2-propylamin;
2-(4-Chlorphenyl)-1-phenyl-2-propylamin;
2-(3,4-Dimethoxyphenyl)-1-phenyl-2-propylamin;
2-(4-Methylphenyl)-1-phenyl-2-propylamin;
2-(4-Methoxyphenyl)-1-phenyl-2-propylamin;
1-(3,4-Dichlorphenyl)-2-phenyl-2-propylamin;
1-(4-Methoxyphenyl)-2-phenyl-2-propylamin;
2-Amino-N-[1-(3-chlorphenyl)-2-phenyl-1-methylethyl]-acetamid;
2-Amino-N-[2-(2-chlorphenyl)-1-phenyl-1-methylethyl]-acetamid;
2-Amino-N-[2-(4-chlorphenyl)-1-phenyl-1-methylethyl]-acetamid;
2-Amino-N-[1-(2-methylphenyl)-2-phenyl-1-methylethyl]-acetamid;
2-Amino-N-[1-(2-chlorphenyl)-2-phenyl-1-methylethyl]-acetamid;
2-Amino-N-[2-(3-chlorphenyl)-1-phenyl-1-methylethyl]-acetamid;
2-Amino-N-[2-(3-aminophenyl)-1-phenyl-1-methylethyl]-acetamid;
2-Amino-N-[2-(3-bromphenyl)-1-phenyl-1-methylethyl]-acetamid;
2-Amino-N-[2-(3-nitrophenyl)-1-phenyl-1-methylethyl]-acetamid;
2-Amino-N-[1-(3-nitrophenyl)-2-phenyl-1-methylethyl]-acetamid;
2-Amino-N-[1-(3-aminophenyl)-2-phenyl-1-methylethyl]-acetamid;
2-Amino-N-[1-(4-chlorphenyl)-2-phenyl-1-methylethyl]-acetamid;
2-Amino-N-[1-(3,4-dichlorphenyl)-2-phenyl-1-methylethyl]-acetamid;
2-Amino-N-[1-(3,4-dimethoxyphenyl)-2-phenyl-1-methylethyl]-acetamid;
2-Amino-N-[2-(3-cyanophenyl)-1-phenyl-1-methylethyl]-acetamid;
2-Amino-N-[1-(4-methylphenyl)-2-phenyl-1-methylethyl]-acetamid;
2-Amino-N-[1-(4-hydroxyphenyl)-2-phenyl-1-methylethyl]-acetamid;
2-Amino-N-[1-(4-hydroxyphenyl)-2-phenylethyl]-acetamid;
2-Amino-N-[2-(4-hydroxyphenyl)-1-phenyl-1-methylethyl]-acetamid;
2-Amino-N-[2-(4-hydroxyphenyl)-1-phenylethyl]-acetamid;
2-Amino-N-[1,2-bis-(4-hydroxyphenyl)-1-methylethyl]-acetamid;
2-Amino-N-[1,2-bis-(4-hydroxyphenyl)-ethyl]-acetamid;
2-Amino-N-[1-(3-methoxyphenyl)-2-phenyl-1-methylethyl]-acetamid;
2-Amino-N-[1-(4-methoxyphenyl)-2-phenyl-1-methylethyl]-acetamid;
2-Amino-N-[2-(3,4-dichlorphenyl)-1-phenyl-1-methylethyl]-acetamid;
2-Amino-N-[2-(4-methoxyphenyl)-1-phenyl-1-methylethyl]-acetamid;
oder ein pharmazeutisch annehmbares Salz hievon.

8. Verfahren zur Herstellung einer pharmazeutischen Zusammensetzung, die eine Verbindung der Formel (IA), wie in Anspruch 2 definiert, oder ein pharmazeutisch annehmbares Salz hievon, in Mischung mit einem pharmazeutisch annehmbaren Adjuvans, Verdünnungsmittel oder Träger umfaßt, welches Verfahren das Mischen der Bestandteile umfaßt.

## Revendications (Revendications pour l'(les) Etat(s) contractant(s) suivant(s): AT, BE, CH, DE, FR, GB, IT, LI, LU, NL, SE)

1. Utilisation d'un composé de formule (I) : 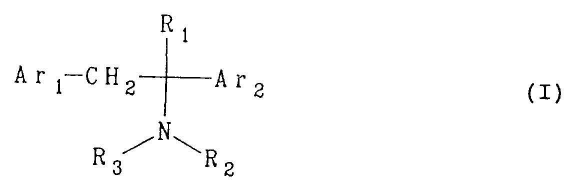 dans lequel :
Ar₁ et Ar₂ représentent indépendamment, un phényle substitué par un ou plusieurs parmi amino, nitro, chlore, brome, hydroxyle, alcoxy en C₁₋₆, alcoyle en C₁₋₆ ou cyano, en outre, l'un des Ar₁ et Ar₂ peut représenter phényle;
R₁ représente hydrogène ou alcoyle en C₁₋₆;
R₂ représente hydrogène ou COCH₂NH₂;
R₃ représente hydrogène ou alcoyle en C₁₋₆; en outre, lorsque R₂ représente hydrogène, alors Ar₁ et Ar₂ peuvent tous deux représenter phényle, un des Ar₁ et Ar₂ ou les deux peuvent représenter fluorophényle ou 2-, 3-, ou 4-pyridinyle, et R₁ peut représenter alcoxycarbonyle en C₁₋₆ ou trifluorométhyle;
pourvu que :
(a) lorsque R₁ et R₂ représentent chacun H et Ar₁ et Ar₂ représentent chacun phényle, alors R₃ est autre que alcoyle en C₁₋₆;
(b) lorsque Ar₂ représente 2-, 3-, ou 4-pyridinyle, alors R₁ représente alcoyle en C₁₋₆, et
(c) lorsque R₁ et R₂ représentent chacun H, alors Ar₁ ne représente pas phényle substitué par amino;
ou un sel pharmaceutiquement acceptable de celui-ci, comme ingrédient actif dans la préparation d'un médicament neuroprotecteur ou anticonvulsivant.

2. Utilisation suivant la revendication 1, dans laquelle le composé de formule (I) est :
la 1,2-diphényléthylamine;
la 1,2-diphényl-2-propylamine;
la 1,2-bis(4-fluorophényl)-2-propylamine;
la 1,2-diphényl-2-butylamine;
la (-)-1,2-diphényl-2-propylamine;
la (+)-1,2-diphényl-2-propylamine;
l'ester méthylique de l'acide 2,3-diphényl-2-aminopropanoïque;
la N-méthyl-1,2-diphényl-2-propylamine;
la 1-(3-nitrophényl)-2-phényl-2-propylamine;
la 1-(3-chlorophényl)-2-phényl-2-propylamine;
la 1-(3-bromophényl)-2-phényl-2-propylamine;
la 1-(3-cyanophényl)-2-phényl-2-propylamine;
la 2-(2-méthylphényl)-1-phényl-2-propylamine;
la 1-(4-chlorophényl)-2-phényl-2-propylamine;
la 1-phényl-2-(3,4-dichlorophényl)-2-propylamine;
la 1-phényl-2-(3-méthoxyphényl)-2-propylamine;
la 1-(4-hydroxyphényl)-2-phényl-2-propylamine;
la 1-(4-hydroxyphényl)-2-phényléthylamine;
la 1-phényl-2-(4-hydroxyphényl)éthylamine;
la 1,2-bis(4-hydroxyphényl)éthylamine;
la 1-phényl-2-(4-hydroxyphényl)-2-propylamine;
la 1,2-bis(4-hydroxyphényl)-2-propylamine;
la 1-phényl-2-(2-pyridinyl)éthylamine;
la 1-phényl-2-(3-pyridinyl)éthylamine;
la 1-phényl-2-(4-pyridinyl)éthylamine;
la 3,3,3-trifluoro-1,2-diphényl-2-propylamine;
la N-méthyl-3,3,3-trifluoro-1,2-diphényl-2-propylamine;
ou un sel pharmaceutiquement acceptable de ceux-ci.

3. 1-Phényl-2-(2-pyridinyl)éthylamine, ou un sel pharmaceutiquement acceptable de celle-ci, à utiliser comme produit pharmaceutique.

4. Composition pharmaceutique comprenant la 1-phényl-2-(2-pyridinyl)éthylamine, ou un sel pharmaceutiquement acceptable de celle-ci, en mélange avec un adjuvant, diluant ou excipient pharmaceutiquement acceptable.

5. Composé de formule (IA) : 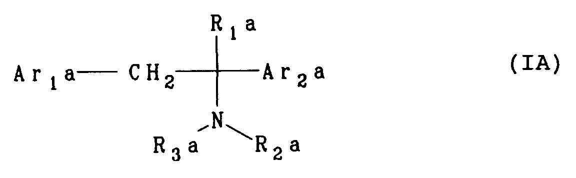 dans lequel :
Ar₁a et Ar₂a représentent indépendamment, un phényle substitué par un ou plusieurs parmi amino, nitro, chlore, brome, hydroxyle, alcoxy en C₁₋₆, alcoyle en C₁₋₆ ou cyano, en outre, l'un des Ar₁a et Ar₂a peut représenter phényle;
R₁a représente hydrogène ou alcoyle en C₁₋₆;
R₂a représente hydrogène ou COCH₂NH₂;
R₃a représente hydrogène ou alcoyle en C₁₋₆;
pourvu que lorsque R₂a représente hydrogène, alors R₁a représente alcoyle en C₁₋₆;
ou un sel pharmaceutiquement acceptable de celui-ci.

6. Composé suivant la revendication 5, dans lequel un ou les deux de Ar₁a et Ar₂a représentent 4-hydroxyphényle.

7. Composé suivant la revendication 5, dans lequel Ar₁a et Ar₂a sont mono- ou bisubstitués.

8. Composé suivant la revendication 5, dans lequel R₁a représente méthyle.

9. Composé suivant la revendication 5, dans lequel R₃a représente hydrogène.

10. Composé suivant la revendication 5, qui est :
la 1-(4-hydroxyphényl)-2-phényl-2-propylamine;
la 1-phényl-2-(4-hydroxyphényl)-2-propylamine;
la 1,2-bis(4-hydroxyphényl)-2-propylamine;
la 1-(3-nitrophényl)-2-phényl-2-propylamine;
la 1-(3-chlorophényl)-2-phényl-2-propylamine;
la 1-(3-bromophényl)-2-phényl-2-propylamine;
la 1-(3-cyanophényl)-2-phényl-2-propylamine;
la 2-(2-méthylphényl)-1-phényl-2-propylamine;
la 1-(4-chlorophényl)-2-phényl-2-propylamine;
la 1-phényl-2-(3,4-dichlorophényl)-2-propylamine;
la 1-phényl-2-(3-méthoxyphényl)-2-propylamine;
la 2-(3-chlorophényl)-1-phényl-2-propylamine;
la 1-(2-chlorophényl)-2-phényl-2-propylamine;
la 2-(2-chlorophényl)-1-phényl-2-propylamine;
la 2-(3-nitrophényl)-1-phényl-2-propylamine;
la 2-(4-chlorophényl)-1-phényl-2-propylamine;
la 2-(3,4-diméthoxyphényl)-1-phényl-2-propylamine;
la 2-(4-méthylphényl)-1-phényl-2-propylamine;
la 2-(4-méthoxyphényl)-1-phényl-2-propylamine;
la 1-(3,4-dichlorophényl)-2-phényl-2-propylamine;
la 1-(4-méthoxyphényl)-2-phényl-2-propylamine;
le 2-amino-N-[1-(3-chlorophényl)-2-phényl-1-méthyléthyl]acétamide;
le 2-amino-N-[2-(2-chlorophényl)-1-phényl-1-méthyléthyl]acétamide;
le 2-amino-N-[2-(4-chlorophényl)-1-phényl-1-méthyléthyl]acétamide;
le 2-amino-N-[1-(2-méthylphényl)-2-phényl-1-méthyléthyl]acétamide;
le 2-amino-N-[1-(2-chlorophényl)-2-phényl-1-méthyléthyl]acétamide;
le 2-amino-N-[2-(3-chlorophényl)-1-phényl-1-méthyléthyl]acétamide;
le 2-amino-N-[2-(3-aminophényl)-1-phényl-1-méthyléthyl]acétamide;
le 2-amino-N-[2-(3-bromophényl)-1-phényl-1-méthyléthyl]acétamide;
le 2-amino-N-[2-(3-nitrophényl)-1-phényl-1-méthyléthyl]acétamide;
le 2-amino-N-[1-(3-nitrophényl)-2-phényl-1-méthyléthyl]acétamide;
le 2-amino-N-[1-(3-aminophényl)-2-phényl-1-méthyléthyl]acétamide;
le 2-amino-N-[1-(4-chlorophényl)-2-phényl-1-méthyléthyl]acétamide;
le 2-amino-N-[1-(3,4-dichlorophényl)-2-phényl-1-méthyléthyl]acétamide;
le 2-amino-N-[1-(3,4-diméthoxyphényl)-2-phényl-1-méthyléthyl]acétamide;
le 2-amino-N-[2-(3-cyanophényl)-1-phényl-1-méthyléthyl]acétamide;
le 2-amino-N-[1-(4-méthylphényl)-2-phényl-1-méthyléthyl]acétamide;
le 2-amino-N-[1-(4-hydroxyphényl)-2-phényl-1-méthyléthyl]acétamide;
le 2-amino-N-[1-(4-hydroxyphényl)-2-phényléthyl]acétamide;
le 2-amino-N-[2-(4-hydroxyphényl)-1-phényl-1-méthyléthyl]acétamide;
le 2-amino-N-[2-(4-hydroxyphényl)-1-phényléthyl]acétamide;
le 2-amino-N-[1,2-bis(4-hydroxyphényl)-1-méthyléthyl]acétamide;
le 2-amino-N-[1,2-bis(4-hydroxyphényl)éthyl]acétamide;
le 2-amino-N-[1-(3-méthoxyphényl)-2-phényl-1-méthyléthyl]acétamide;
le 2-amino-N-[1-(4-méthoxyphényl)-2-phényl-1-méthyléthyl]acétamide;
le 2-amino-N-[2-(3,4-dichlorophényl)-1-phényl-1-méthyléthyl]acétamide;
le 2-amino-N-[2-(4-méthoxyphényl)-1-phényl-1-méthyléthyl]acétamide;
ou un sel pharmaceutiquement acceptable de ceux-ci.

11. Composé de formule (IA) tel que défini à la revendication 5, ou un sel pharmaceutiquement acceptable de celui-ci, à utiliser comme produit pharmaceutique.

12. Composition pharmaceutique comprenant un composé de formule (IA) tel que défini à la revendication 5, ou un sel pharmaceutiquement acceptable de celui-ci, en mélange avec un adjuvant, diluant ou excipient pharmaceutiquement acceptable.

13. Procédé de préparation d'un composé de formule (IA) tel que défini à la revendication 5, ou un sel pharmaceutiquement acceptable de celui-ci, qui comprend :
a) pour les composés dans lesquels R₂a représente hydrogène et R₃a représente alcoyle en C₁₋₆, l'alcoylation d'un composé correspondant de formule (IA) dans lequel R₃a représente hydrogène;
b) pour les composés dans lesquels R₂a et R₃a représentent tous deux hydrogène, hydrolyse d'amide d'un composé correspondant de formule (IIA) : 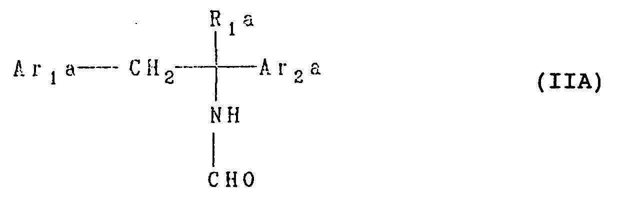 dans lequel Ar₁a, Ar₂a et R₁a sont tels que définis à la revendication 5;
c) pour les composés dans lesquels R₂a et R₃a représentent tous deux hydrogène, clivage réducteur de carbamate d'un composé de formule (IIIA) : 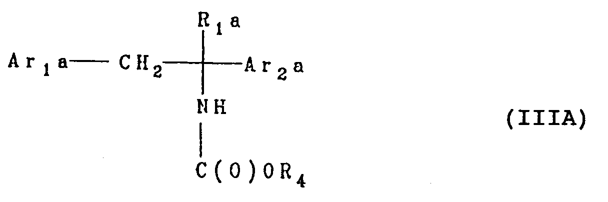 dans lequel Ar₁a, Ar₂a et R₁a sont tels que définis à la revendication 5, et R₄ représente un radical alcoyle ou aryle;
d) pour les composés dans lesquels R₂a représente COCH₂NH₂, élimination du groupe protecteur de l'amine d'un composé de formule (IVA) : 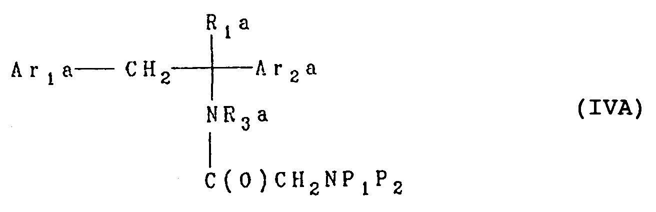 dans lequel P₁ et P₂ constituent ensemble un groupe protecteur approprié, et Ar₁a, Ar₂a, R₁a et R₃a sont tels que définis à la revendication 5, ou
e) pour les composés dans lesquels R₂a représente COCH₂NH₂, amination d'un composé de formule (VA) : 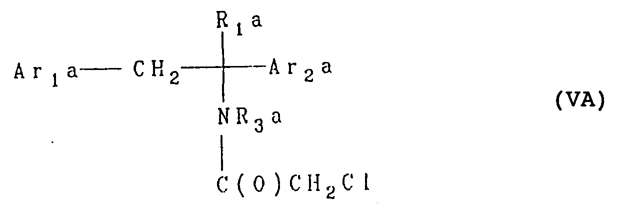 dans lequel Ar₁a, Ar₂a, R₁a et R₃a sont tels que définis à la revendication 5.

## Revendications (Revendications pour l'(les) Etat(s) contractant(s) suivant(s): ES, GR)

1. Procédé de préparation d'une composition pharmaceutique comprenant de la 1-phenyl-2-(2-pyridinyl)éthylamine, ou un sel pharmaceutiquement acceptable de celle-ci, en mélange avec un adjuvant, diluant ou excipient pharmaceutiquement acceptable, qui comprend le mélange des ingrédients.

2. Procédé de préparation d'un composé de formule (IA) : 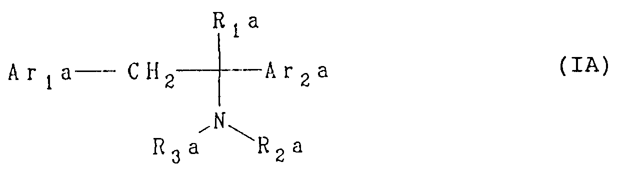 dans lequel :
Ar₁a et Ar₂a représentent indépendamment, un phényle substitué par un ou plusieurs parmi amino, nitro, chlore, brome, hydroxyle, alcoxy en C₁₋₆, alcoyle en C₁₋₆ ou cyano, en outre, l'un des Ar₁a et Ar₂a peut représenter phényle;
R₁a représente hydrogène ou alcoyle en C₁₋₆;
R₂a représente hydrogène ou COCH₂NH₂;
R₃a représente hydrogène ou alcoyle en C₁₋₆; pourvu que lorsque R₂a représente hydrogène, alors R₁a représente alcoyle en C₁₋₆;
ou un sel pharmaceutiquement acceptable de celui-ci; qui comprend :
a) pour les composés dans lesquels R₂a représente hydrogène et R₃a représente alcoyle en C₁₋₆, l'alcoylation d'un composé correspondant de formule (IA) dans lequel R₃a représente hydrogène;
b) pour les composés dans lesquels R₂a et R₃a représentent tous deux hydrogène, hydrolyse d'amide d'un composé correspondant de formule (IIA) : 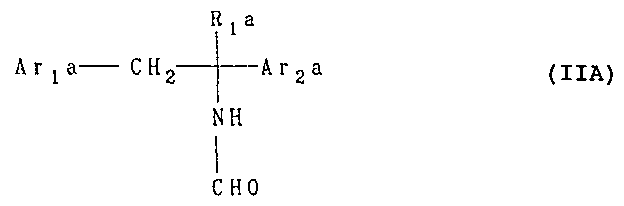 dans lequel Ar₁a, Ar₂a et R₁a sont tels que définis ci-dessus;
c) pour les composés dans lesquels R₂a et R₃a représentent tous deux hydrogène, clivage réducteur de carbamate d'un composé de formule (IIIA) : 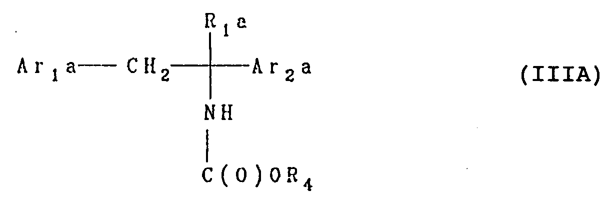 dans lequel Ar₁a, Ar₂a et R₁a sont tels que définis ci-dessus, et R₄ représente un radical alcoyle ou aryle;
d) pour les composés dans lesquels R₂a représente COCH₂NH₂, élimination du groupe protecteur de l'amine d'un composé de formule (IVA) : 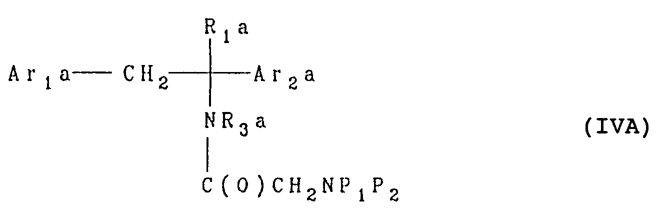 dans lequel P₁ et P₂ constituent ensemble un groupe protecteur approprié, et Ar₁a, Ar₂a, R₁a et R₃a sont tels que définis ci-dessus, ou
e) pour les composés dans lesquels R₂a représente COCH₂NH₂, amination d'un composé de formule (VA) : 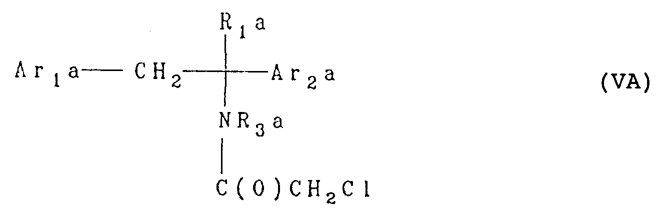 dans lequel Ar₁a, Ar₂a, R₁a et R₃a sont tels que définis ci-dessus.

3. Procédé suivant la revendication 2, dans lequel un ou les deux de Ar₁a et Ar₂a représentent 4-hydroxyphényle.

4. Procédé suivant la revendication 2, dans lequel Ar₁a et Ar₂a sont mono- ou bisubstitués.

5. Procédé suivant la revendication 2, dans lequel R₁a représente méthyle.

6. Procédé suivant la revendication 2, dans lequel R₃a représente hydrogène.

7. Procédé suivant la revendication 2, dans lequel le composé de formule (IA) est :
la 1-(4-hydroxyphényl)-2-phényl-2-propylamine;
la 1-phényl-2-(4-hydroxyphényl)-2-propylamine;
la 1,2-bis(4-hydroxyphényl)-2-propylamine;
la 1-(3-nitrophényl)-2-phényl-2-propylamine;
la 1-(3-chlorophényl)-2-phényl-2-propylamine;
la 1-(3-bromophényl)-2-phényl-2-propylamine;
la 1-(3-cyanophényl)-2-phényl-2-propylamine;
la 2-(2-méthylphényl)-1-phényl-2-propylamine;
la 1-(4-chlorophényl)-2-phényl-2-propylamine;
la 1-phényl-2-(3,4-dichlorophényl)-2-propylamine;
la 1-phényl-2-(3-méthoxyphényl)-2-propylamine;
la 2-(3-chlorophényl)-1-phényl-2-propylamine;
la 1-(2-chlorophényl)-2-phényl-2-propylamine;
la 2-(2-chlorophényl)-1-phényl-2-propylamine;
la 2-(3-nitrophényl)-1-phényl-2-propylamine;
la 2-(4-chlorophényl)-1-phényl-2-propylamine;
la 2-(3,4-diméthoxyphényl)-1-phényl-2-propylamine;
la 2-(4-méthylphényl)-1-phényl-2-propylamine;
la 2-(4-méthoxyphényl)-1-phényl-2-propylamine;
la 1-(3,4-dichlorophényl)-2-phényl-2-propylamine;
la 1-(4-méthoxyphényl)-2-phényl-2-propylamine;
le 2-amino-N-[1-(3-chlorophényl)-2-phényl-1-méthyléthyl]acétamide;
le 2-amino-N-[2-(2-chlorophényl)-1-phényl-1-méthyléthyl]acétamide;
le 2-amino-N-[2-(4-chlorophényl)-1-phényl-1-méthyléthyl]acétamide;
le 2-amino-N-[1-(2-méthylphényl)-2-phényl-1-méthyléthyl]acétamide;
le 2-amino-N-[1-(2-chlorophényl)-2-phényl-1-méthyléthyl]acétamide;
le 2-amino-N-[2-(3-chlorophényl)-1-phényl-1-méthyléthyl]acétamide;
le 2-amino-N-[2-(3-aminophényl)-1-phényl-1-méthyléthyl]acétamide;
le 2-amino-N-[2-(3-bromophényl)-1-phényl-1-méthyléthyl]acétamide;
le 2-amino-N-[2-(3-nitrophényl)-1-phényl-1-méthyléthyl]acétamide;
le 2-amino-N-[1-(3-nitrophényl)-2-phényl-1-méthyléthyl]acétamide;
le 2-amino-N-[1-(3-aminophényl)-2-phényl-1-méthyléthyl]acétamide;
le 2-amino-N-[1-(4-chlorophényl)-2-phényl-1-méthyléthyl]acétamide;
le 2-amino-N-[1-(3,4-dichlorophényl)-2-phényl-1-méthyléthyl]acétamide;
le 2-amino-N-[1-(3,4-diméthoxyphényl)-2-phényl-1-méthyléthyl]acétamide;
le 2-amino-N-[2-(3-cyanophényl)-1-phényl-1-méthyléthyl]acétamide;
le 2-amino-N-[1-(4-méthylphényl)-2-phényl-1-méthyléthyl]acétamide;
le 2-amino-N-[1-(4-hydroxyphényl)-2-phényl-1-méthyléthyl]acétamide;
le 2-amino-N-[1-(4-hydroxyphényl)-2-phényléthyl]acétamide;
le 2-amino-N-[2-(4-hydroxyphényl)-1-phényl-1-méthyléthyl]acétamide;
le 2-amino-N-[2-(4-hydroxyphényl)-1-phényléthyl]acétamide;
le 2-amino-N-[1,2-bis(4-hydroxyphényl)-1-méthyléthyl]acétamide;
le 2-amino-N-[1,2-bis(4-hydroxyphényl)éthyl]acétamide;
le 2-amino-N-[1-(3-méthoxyphényl)-2-phényléthyl]acétamide;
le 2-amino-N-[1-(4-méthoxyphényl)-2-phényléthyl]acétamide;
le 2-amino-N-[2-(3,4-dichlorophényl)-1-phényléthyl]acétamide;
le 2-amino-N-[2-(4-méthoxyphényl)-1-phényléthyl]acétamide;
ou un sel pharmaceutiquement acceptable de ceux-ci.

8. Procédé de préparation d'une composition pharmaceutique comprenant un composé de formule (IA) tel que défini à la revendication 2, ou un sel pharmaceutiquement acceptable de celui-ci, en mélange avec un adjuvant, diluant ou excipient pharmaceutiquement acceptable; qui comprend le mélange des ingrédients.
